(19) Europäisches Patentamt
European Patent Office
Office européen des brevets

(11) EP 1 495 748 A1

(12) **DEMANDE DE BREVET EUROPEEN**

(43) Date de publication:
**12.01.2005 Bulletin 2005/02**

(51) Int Cl.⁷: **A61K 7/043**, A61K 7/04, C09D 101/10, C09D 101/18

(21) Numéro de dépôt: **04103198.0**

(22) Date de dépôt: **06.07.2004**

(84) Etats contractants désignés:
**AT BE BG CH CY CZ DE DK EE ES FI FR GB GR HU IE IT LI LU MC NL PL PT RO SE SI SK TR**
Etats d'extension désignés:
**AL HR LT LV MK**

(30) Priorité: **07.07.2003 FR 0350299**

(71) Demandeur: **L'OREAL**
**75008 Paris (FR)**

(72) Inventeurs:
- **Ilekti, Philippe**
**75003 Paris (FR)**
- **Mondet, Jean**
**93600 Aulnay Sous Bois (FR)**
- **Tournilhac, Florence**
**75011 Paris (FR)**

(74) Mandataire: **Poulin, Gérard**
**BREVALEX**
**3, rue du Docteur Lancereaux**
**75008 Paris (FR)**

(54) **Utilisation de derives cellulosiques modifies en tant qu'agent epaississant de la phase organique d'une composition de vernis a ongles**

(57) L'invention a trait à l'utilisation de certains dérivés cellulosiques particuliers choisis parmi les esters de cellulose et les nitrocelluloses, lesdits dérivés comportant des fonctions hydroxyles libres remplacées par des radicaux de formule -OYR, en tant qu'agents épaissisants de la phase organique d'une composition de vernis à ongles, notamment lorsque ladite phase organique comprenant au moins un solvant ester.

EP 1 495 748 A1

## Description

### DOMAINE TECHNIQUE

La présente invention a trait à l'utilisation de nouveaux dérivés cellulosiques en tant qu'agents épaississants de la phase organique d'une composition de vernis à ongles, en particulier lorsque ladite phase organique comprend des solvants esters.

La présente invention a trait également à des compositions de vernis à ongles comprenant certains de ces dérivés cellulosiques.

### ETAT DE LA TECHNIQUE ANTERIEURE

Dans les compositions de vernis à ongles, il est courant d'utiliser des agents épaississants, en vue d'épaissir la phase organique de ces compositions. L'épaississement de la phase organique permet notamment d'améliorer la suspension des pigments présents dans cette phase, tels que les pigments de dioxyde de titane et d'éviter la sédimentation de ceux-ci lors d'un stockage. L'épaississement de la phase organique facilite également la prise de la composition hors de son conditionnement et la répartition de ladite composition sur la zone à traiter.

Pour épaissir les compositions de vernis à ongles, il est utilisé couramment des argiles telles que des bentones. Les bentones sont des particules d'argile, plus particulièrement des particules d'hectorite modifiées en surface par des chlorures d'alkyle ammonium quaternaire. Toutefois, bien qu'elles apportent aux compositions dans lesquelles elles sont incorporées un caractère rhéofluidifiant et un comportement thixotrope satisfaisants, les bentones sont toutefois difficiles à mettre en oeuvre et nécessitent notamment une étape de mise en dispersion. Cette étape de mise en dispersion nécessite beaucoup d'énergie mécanique pour mouiller et pour désagglomérer les particules de la bentone, afin d'obtenir un gel homogène contenant un minimum de défauts. En outre, la présence de bentones dans des compositions filmogènes, telles que les vernis à ongles, modifie les propriétés du film obtenu après l'application de la composition, et contribue notamment à diminuer la résistance aux chocs du film et à augmenter l'écaillage dudit film. Enfin, le fait que les bentones se présentent sous forme de particules de tailles de l'ordre du micron, engendre une matification du film formé et ainsi une perte de brillance importance de ce film.

Il serait donc souhaitable de disposer de nouveaux agents épaississants ne présentant pas les inconvénients susmentionnés et en particulier ne contribuant pas à amenuiser la brillance et la résistance du film déposé.

Ainsi, les auteurs ont découvert, de façon surprenante, qu'en incorporant dans une composition de vernis à ongles, certains dérivés de cellulose particuliers, il était possible d'obtenir une composition, qui présente d'excellentes propriétés rhéofluidifiantes et thixotropiques, une viscosité en absence de cisaillement suffisamment forte pour éviter la sédimentation des sédiments contenus dans cette composition, et qui, après application, donne des films ne présentant plus la matité inhérente aux films obtenus à partir de compositions comprenant des argiles en tant qu'agents épaississants.

### EXPOSE DE L'INVENTION

Ainsi, l'invention a pour objet l'utilisation en tant qu'agent épaississant de la phase organique d'une composition de vernis à ongles de dérivés cellulosiques choisis parmi les nitrocelluloses, les esters de cellulose, lesdits dérivés comportant des fonctions hydroxyles libres remplacées, en tout ou partie, par des radicaux de formule -OYR, dans laquelle :

- R représente un groupe choisi parmi :

  a) les groupes hydrocarbonés à chaînes linéaires ou ramifiées, saturés ou insaturés, ou cycliques, saturés ou insaturés,
  lesdits groupes pouvant comporter dans leurs chaînes un ou plusieurs groupes aromatiques et/ou un ou plusieurs hétéroatomes choisis parmi O, N, P, Si, S ;
  b) les groupes fluoro- ou perfluoroalkyles ;
  c) les groupes de nature polymérique choisis parmi les polyoléfines, les polydiènes, les polycondensats ;
  d) les groupes organosiloxanes ou polyorganosiloxanes ;
  e) les groupes mésogènes

  lesdits groupes répondant à la définition a), b), c), d) ou e) pouvant comporter au moins un groupement apte à établir une liaison hydrogène,
- Y représente une liaison simple ou un groupe de liaison divalent.

La présente invention a également pour objet une composition de vernis à ongles comprenant une phase organique à base d'au moins un solvant organique et comprenant au moins un dérivé cellulosique comprenant des fonctions hydroxyles remplacées en tout ou partie par des radicaux de formule -OYR, dans laquelle :

- R représente un groupe choisi parmi :

  a) les groupes hydrocarbonés à chaînes linéaires ou ramifiées, saturés ou insaturés, ou cycliques, saturés ou insaturés,
  lesdits groupes pouvant comporter dans leurs chaînes un ou plusieurs groupes aromatiques et/ou un ou plusieurs hétéroatomes choisis parmi O, N, P, Si, S ;
  b) les groupes fluoro- ou perfluoroalkyles ;
  c) les groupes de nature polymérique choisis parmi les polyoléfines, les polydiènes, les polycondensats ;
  d) les groupes organosiloxanes ou polyorganosiloxanes ;
  e) les groupes mésogènes;

  lesdits groupes répondant à la définition a), b), c), d) ou e) pouvant comporter au moins un groupement apte à établir une liaison hydrogène,
- Y représente une liaison simple ou un groupe de liaison divalent,

ledit dérivé cellulosique étant un ester de cellulose lorsque R répond à la définition a), c) et d), un ester de cellulose ou une nitrocellulose lorsque R répond à la définition b), e).

De préférence, pour les compositions de l'invention, lorsque le dérivé de cellulose est un ester de cellulose, R est choisi parmi les groupes répondant aux définitions a), b), c) et e) données ci-dessus.

On précise que, par liaison simple, on entend, dans ce qui précède et ce qui suit, une liaison covalente simple formant un pont entre l'oxygène et le groupe R. Dans ce cas, le groupe -OYR correspond à un groupe -OR.

On précise que, par groupe de liaison divalent, on entend, dans ce qui précède et ce qui suit, un groupe organique espaceur formant pont entre l'atome d'oxygène et le groupe R, lesdits groupes de liaison pouvant être choisi parmi les groupes -(C=O)-, - (C=O) O-, $-SO_2-$, -CO-NH- ou -CO-NR'-,$-Si(R_3)_2-$, les $R_3$ identiques ou différents, étant un groupe hydrocarboné, linéaire ou ramifié comportant de 1 à 500 atomes de carbone, ou cyclique comportant de 3 à 500 atomes de carbone, ledit groupe étant saturée ou insaturée et pouvant comporter un ou plusieurs hétéroatomes choisis parmi O, N, S, Si et/ou P et R' désignant un radical alkyle en C1 à C4. De préférence, $R_3$ représente un groupe alkyle comprenant de 1 à 10 atomes de carbone.

On précise que, par nitrocellulose, on entend, dans ce qui précède et ce qui suit, un polymère constitué par un enchaînement en $\alpha$ (1-4) de cycles anhydroglucose nitrés en partie obtenu par estérification d'une partie des fonctions hydroxyles libres d'une cellulose, par exemple, par action de l'acide nitrique en présence d'acide sulfurique.

On précise que, par 'ester de cellulose', on entend, dans ce qui précède et ce qui suit, un polymère constitué par un enchaînement en $\alpha$ (1-4) de cycles anhydroglucose estérifiés en partie, l'estérification étant obtenue par réaction d'une partie des fonctions hydroxyles libres desdits cycles avec un acide carboxylique ou un dérivé d'acide carboxylique (chlorure d'acide, anhydride d'acide). Avantageusement, les esters de cellulose peuvent être des acétates, des propionates, des butyrates, des isobutyrates, des phtalates, des acétobutyrates, des acétopropionates de cellulose.

De préférence, la phase organique de la composition de vernis selon l'invention, comprend au moins un solvant ester, de préférence un solvant ester comprenant de 3 à 8 atomes de carbone, en particulier les esters à chaîne courte (ayant de 3 à 8 atomes de carbone au total) tels que l'acétate d'éthyle, l'acétate de méthyle, l'acétate de propyle, l'acétate de n-butyle, l'acétate d'isobutyle, l'acétate d'isopentyle et leurs mélanges.

L'intérêt des dérivés de cellulose utilisés dans le cadre de l'invention réside dans le fait qu'ils présentent une excellente solubilité dans la phase organique des compositions de vernis à ongle (du fait de la présence de groupes nitro- ou ester) telle que définie précédemment et qu'ils produisent, grâce aux groupes R susmentionnés, un épaississement de ladite phase organique desdites compositions de vernis à ongles et empêchent ainsi une sédimentation des pigments contenus dans les compositions de vernis à ongles de l'invention. De plus, de tels dérivés engendrent, après application sur un substrat kératinique de la composition les contenant, des films brillants et résistants notamment à l'écaillage.

Selon l'invention, les groupes R peuvent être des groupes répondant à la définition a) donnée ci-dessus, à savoir ils peuvent être un groupe hydrocarboné, saturé ou insaturé, à chaîne linéaire ou ramifiée, pouvant comporter de 1 à 50 atomes de carbone, de préférence de 8 à 50 atomes de carbone et pouvant comporter dans sa chaîne un ou plusieurs groupes aromatiques et/ou un ou plusieurs hétéroatomes choisis parmi O, N, P, Si, S, ou peuvent être des groupes hydrocarbonés, saturés ou insaturés, cycliques, pouvant comporter de 3 à 50 atomes de carbone. On précise que ces groupes sont de nature non polymérique, c'est-à-dire qu'ils ne résultent pas de la polymérisation ou polycondensation d'un ou plusieurs monomères.

Des groupes appropriés particulièrement avantageux répondant à cette définition peuvent être des groupes alkyles linéaires ou ramifiés comportant de 1 à 50 atomes de carbone, de préférence de 8 à 50 atomes de carbone, et comportant éventuellement un ou plusieurs hétéroatomes choisis parmi O, N, P, Si, S.

Des groupes appropriés peuvent être également des groupes cycloalkyles (par exemple, mono- ou polycycliques) comportant de 3 à 50 atomes de carbone.

Selon l'invention, les groupes R peuvent être des groupes fluoro- ou perfluoroalkyles (définition b)), c'est-à-dire des groupes alkyles, dont tous ou partie (tous lorsque le groupe est perfluoro) des atomes d'hydrogène sont substitués par des atomes de fluor. De préférence, ces groupes fluoro- ou perfluoroalkyles comprennent de 6 à 50 atomes de carbone.

Selon l'invention, les groupes R peuvent être également des groupes de nature polymérique choisis parmi les polyoléfines, les polydiènes, les polycondensats (définition c)).

Des polyoléfines appropriées peuvent être du polyéthylène, comprenant, avantageusement, de 10 à 500 motifs $-CH_2-$, des copolymères choisis parmi les copolymères suivants :

- les copolymères (éthylène/propylène) ;
- les copolymères (éthylène/butène) ;
- les copolymères (éthylène/hexène) ;
- les copolymères (éthylène/octène).

Des exemples de polydiènes sont les polybutadiènes, le polyisoprène, de préférence hydrogénés.

Selon l'invention, les polycondensats peuvent être des polyesters, des polyamides, des polyesteramides, des polyuréthannes, des polycarbonates, des polyurées, des copolymères (urée/uréthanne), des polyéthers.

A titre d'exemples de polyesters, on peut citer ceux issus de la polyestérification entre des diols tels que l'éthylène glycol, le propylène glycol, le diéthylène glycol, le néopentyl glycol, le 1,4-butanediol, le furanne diméthanol, le cyclohexane diméthanol, le glycérol, le triméthylolpropane, le pentaérythitol et leurs mélanges, avec des acides polycarboxyliques, en particulier des acides carboxyliques et leurs dérivés esters en $C_1$-$C_4$, par exemple, l'acide succinique, glutarique et adipique ou leurs esters de diméthyle, l'anhydride phtalique ou le téréphtalate de diméthyle ou avec des lactones, par exemple la caprolactone.

A titre d'exemples de polyesteramides, on peut citer ceux obtenus par ajout d'aminoalcools tels que l'éthanolamine dans des mélanges de polyestérification, tels que ceux mentionnés ci-dessus.

A titre d'exemples de polycarbonates, on peut citer ceux obtenus par réaction de diols tels que le 1,3-propanediol, le 1,4-butanediol, le 1,8-hexanediol, le diéthylèneglycol, le tétraéthylèneglycol avec des carbonates de diaryle, de diacyle, ou aliphatiques par exemple le carbonate de diphényle, ou avec du phosgène.

A titre d'exemples de polyamides, on peut citer ceux obtenus par condensation entre un diacide carboxylique (ou dérivé ester en $C_1$-$C_4$) aliphatique, cycloaliphatique ou aromatique en $C_3$-$C_{50}$ et une diamine en $C_2$-$C_{50}$ aliphatique, linéaire ou ramifiée, cycloaliphatique ou aromatique, les diacides pouvant être choisis parmi les diacides mentionnés ci-dessus avec en plus les acides gras dimères (provenant de la condensation entre deux molécules de mono acides gras insaturés), les diamines pouvant être choisies parmi l'éthylènediamine, le 1,2-diaminopropane, le 1,3-diaminopropane, le 1,4-diaminobutane, le 1,2-diamino-2-méthylpropane, le 1,6-diaminohexane, le 1,10-diaminodécane, l'isophoronediamine, l'adamantanediamine, la 2,6-diaminopyridine.

A titre d'exemples de polyuréthannes, polyurées et polyurées-uréthannes, on peut citer ceux obtenus par polyaddition entre des diisocyanates aliphatiques, cycloaliphatiques et/ou aromatiques de $C_4$-$C_{100,}$ de préférence $C_4$-$C_{30}$ tels que l'hexaméthylène diisocyanate, l'isophorone diisocyanate, le toluène diisocyanate, le diphénylméthane diisocyanate et des diols tels que définis ci-dessus, ou des diamines telles que définies ci-dessus, ou des mélanges diols/diamines.

A titre d'exemples de polyéthers, on peut citer les copolymères entre oxyéthylène et oxypropylène, le polytétraméthylèneoxyde.

Pour les compositions de l'invention, lorsque le dérivé de cellulose est un ester de cellulose et lorsque Y représente un groupe -CO-, alors les groupes R répondant aux définitions a) ou c) sont, de préférence, différents de ceux préexistants sur l'ester de cellulose de base, avant remplacement, en tout ou partie, des fonctions hydroxyles libres par des radicaux de formule -OYR (ces groupes préexistants étant des groupes hydrocarbonés, par exemple, tels que l'ester de cellulose de base, avant greffage des groupes R tels que définis ci-dessus, soit un acétate, propionate, butyrate, isobutyrate, phtalate, acétobutyrate ou acétopropionate de cellulose).

Les groupes R peuvent être également des groupes organosiloxanes ou polyorganosiloxanes (définition d)). De tels groupes peuvent répondre à la formule suivante :

$$- [Si(Ra,Rb)-O-]_p-Si\ (Ra, Rb, Rc)$$

dans laquelle :

les Ra, Rb et Rc, identiques ou différents représentent une chaîne alkyle linéaire ou ramifiée, comportant de 1 à 20 atomes de carbone, un phényle ou une chaîne perfluoroalkyle comportant de 3 à 20 atomes de carbone et p étant un entier allant de 0 à 500.

Enfin, les groupes R peuvent représenter des groupes mésogènes, c'est-à-dire des groupes aptes à donner des phases cristaux liquides.

Parmi les groupes mésogènes répondant à la définition donnée ci-dessus susceptibles de donner des phases cristaux liquides provoquant la gélification, on peut citer des groupes alkylaryles, alkylcycloalkyles, alcoxyaryles, alcoxycycloalkyles.

Avantageusement, de tels groupes répondant à la définition donnée ci-dessus peuvent répondre aux formules suivantes :

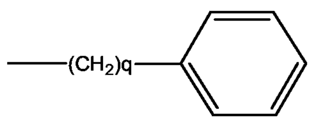
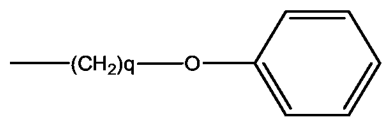

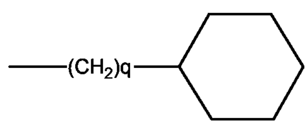
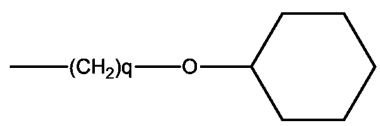

avec q étant un entier allant de 1 à 10.

Parmi les groupes mésogènes répondant à la définition donnée ci-dessus susceptibles de convenir, on peut citer avantageusement les groupes représentés par la formule générale suivante :

$$-E-F-E-G,$$

dans laquelle :

- les E, identiques ou différents, sont des groupes divalents aromatiques tels que :

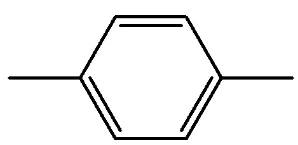
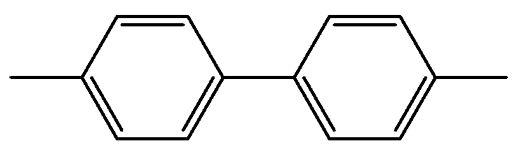

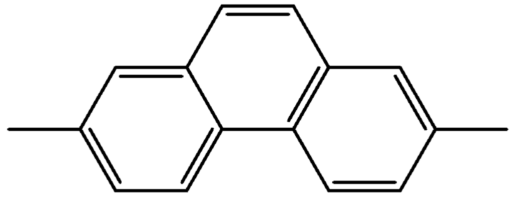
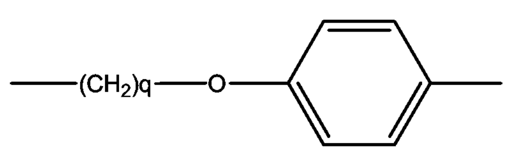

  avec q représentant un entier allant de 1 à 10 ;
- F est un groupe divalent insaturé et non cyclique tel que :
  -C=C-, -N=N-, -CH=N-, -CO-O-

ou une liaison simple;

- G représente un groupe terminal de formules suivantes:

$$-O-Ru, -CO-Ru, -CN, -COOH, -(CH_2)_q-Ru$$

avec Ru représentant un groupe alkyle linéaire ou ramifié comportant de 1 à 10 atomes de carbone et q représentant un entier allant de 1 à 10.

Des groupes particuliers répondant à cette définition sont les groupes de formules suivantes :

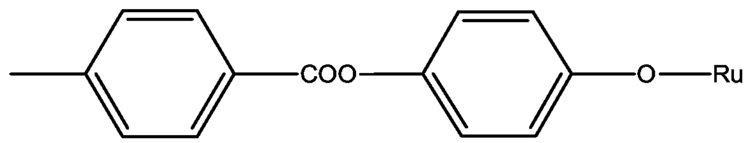

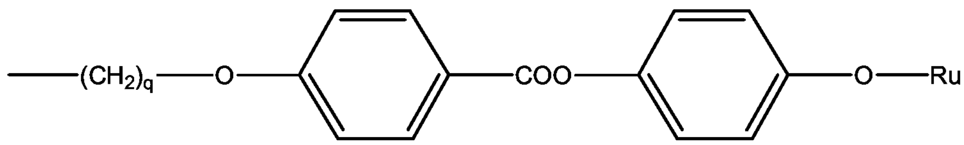

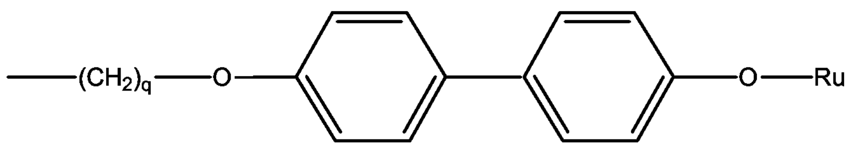

Parmi les groupes mésogènes susceptibles de convenir, on peut citer également les dérivés de l'acide parahydroxybenzoïque répondant à l'une des formules suivantes :

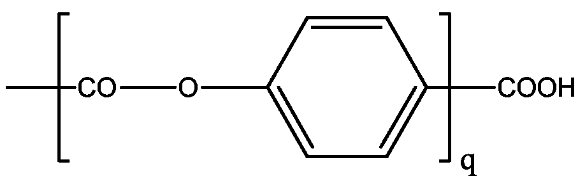
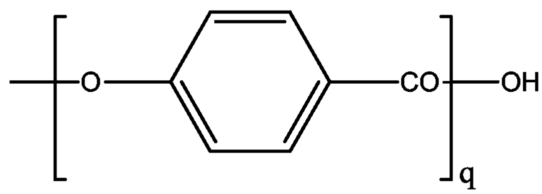

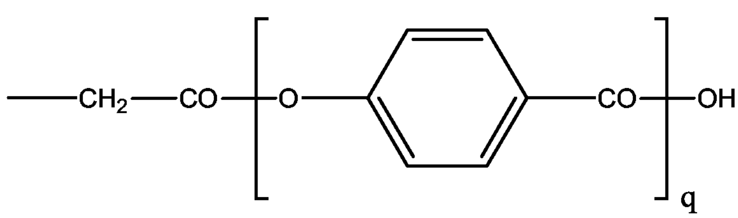

avec q ayant la même définition que celle donnée précédemment.

Enfin, on peut citer comme groupe mésogène avantageux, des groupes dérivés du cholestérol tel que celui représenté par la formule suivante :

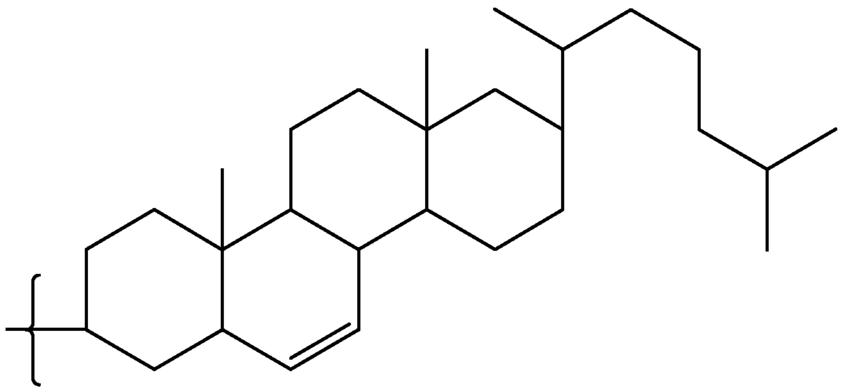

la liaison avec Y ou O (lorsque Y représente une liaison simple) se faisant au niveau de la liaison interrompue par l'accolade.

Avantageusement, les groupes répondant aux définitions a), b), c), d) et e) peuvent être porteurs d'un ou plusieurs groupements aptes à établir une liaison hydrogène.

On précise que, par groupement apte à établir une liaison hydrogène, on entend un groupement comportant soit un atome d'hydrogène lié à un atome électronégatif, soit un atome électronégatif. Lorsque le groupement comporte un atome d'hydrogène lié à un atome électronégatif, l'atome d'hydrogène peut interagir avec un autre atome électronégatif porté, par exemple par une autre molécule, telle que la kératine de l'ongle, pour former une liaison hydrogène. Lorsque le groupement comporte un atome électronégatif, l'atome électronégatif peut interagir avec un atome d'hydrogène lié à un atome électronégatif porté, par exemple par une autre molécule, telle que la kératine de l'ongle, pour former une liaison hydrogène.

Avantageusement, ces groupements aptes à établir une liaison hydrogène peuvent être des groupes choisis parmi les groupes suivants :

- hydroxyle -OH ;
- acide carboxylique -COOH ;
- amino $-NR_1R_2$ avec $R_1$ et $R_2$, identiques ou différents ;
- pyridino de formule :

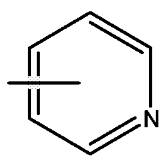

- pyrimidino de formule :

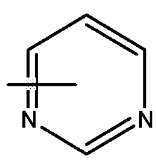

- oxazolino répondant à l'une des formules suivantes :

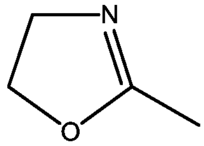
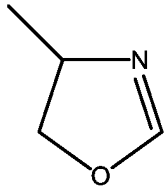
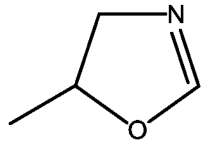

- amido de formules -NH-CO-R' ou -CO-NH-$R_1$;
- pyrrolidono répondant à l'une des formules suivantes :

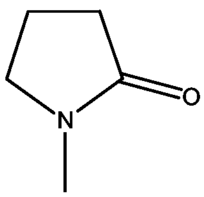
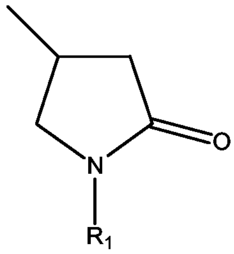
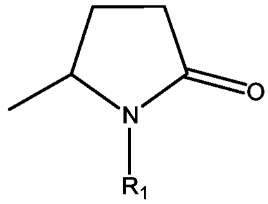
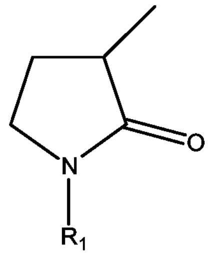

- carbamoyl de formules -O-CO-NH-R' ou -NH-CO-O-R' ;
- thiocarbamoyl de formules -O-CS-$NHR_1$ ou -NH-CS-O-R' ;
- carbonato -O-CO-O-R';
- uréyl-$NR_1$-CO-N $(R_1)_2$, les $R_1$ étant identiques ou différents ;
- thiouréyl -$NR_1$-CS-N$(R_1)_2$, les $R_1$ étant identiques ou différents ;
- oxamido -$NR_1$-CO-CO-N$(R_1)_2$ avec les $R_1$ identiques ou différents ;
- guanidino -NH-C(=NH)-N$(R_1)$2 avec les $R_1$ identiques ou différents ;
- biguanidino -NH-C(=NH)-NH-C(=NH)-N$(R_1)_2$ avec les Ri identiques ou différents ;
- sulfonamido -$NR_1$-S$(=O)_2$-R',

avec $R_1$ et $R_2$ représentant H ou un groupe alkyle comprenant de 1 à 4 atomes de carbone, R' représentant un radical alkyle comprenant de 1 à 4 atomes de carbone.

Il est entendu que de tels groupements sont portés par la chaîne R soit en bout de chaîne soit latéralement à ladite chaîne.

Des dérivés porteurs d'au moins un groupement apte à établir une liaison hydrogène sont particulièrement avantageux, car ils apportent aux compositions de vernis à ongles les contenant des propriétés adhérentes très élevées, grâce à l'aptitude de ces groupements à établir une liaison hydrogène, par exemple, avec la kératine de l'ongle.

De préférence, les groupes R répondant aux définitions a), b), c), d) ou e) ne sont pas chargés, c'est-à-dire ne sont pas de nature anionique, cationique, amphotère (c'est-à-dire porteur à la fois d'une charge négative et d'une charge positive) ou bétaïne.

Comme cela a été mentionné plus haut, les groupes R greffés sur les fonctions hydroxyles des dérivés de cellulose, confèrent à ces dérivés des propriétés épaississantes, en particulier lorsque ces dérivés sont incorporés dans des compositions de vernis à ongles comprenant une phase organique, en particulier une phase organique à base de solvant(s) ester(s).

Le mécanisme responsable de l'effet épaississant varie selon la nature des groupes R greffés sur les dérivés de cellulose.

Ainsi, un premier mécanisme peut consister en une séparation de phase entre les groupes R et le squelette des dérivés de cellulose. La séparation de phase est due à une incompatibilité (insolubilité) entre le squelette des dérivés cellulosiques porteurs des groupes esters ou nitrates (plutôt polaires) et les groupes R latéraux (très apolaires dans le cas des organosiloxanes) (les groupes latéraux greffés sont à la fois incompatibles avec le squelette des dérivés cellulosiques et avec le solvant). Ce mécanisme se rencontre dans le cas où R représente un groupe organosiloxane ou polyorganosiloxane, telles que ceux de formules suivantes :

$$- [Si(Ra,Rb)-O-]_p-Si\ (Ra, Rb, Rc)$$

dans laquelle :

les Ra, Rb et Rc, identiques ou différents représentent une chaîne alkyle linéaire ou ramifiée, comportant de 1 à 20 atomes de carbone, un phényle ou une chaîne perfluoroalkyle comportant de 3 à 20 atomes de carbone et p étant un entier allant de 0 à 500.

Un second mécanisme responsable de l'effet épaississant des dérivés de cellulose peut consister en une cristallisation des groupements portés par R en présence, par exemple, des solvants esters de la phase organique des compositions de vernis à ongles.

Un tel mécanisme peut être le cas :

- des greffons polyoléfines, tels que le polyéthylène, les copolymères (éthylène/propylène), (éthylène/butène), (éthylène/héxène), (éthylène/octène) ;
- les groupes perfluoroalkyles linéaires comportant de 6 à 30 atomes de carbone ;
- les greffons polyesters, tels que les polyesters aliphatiques comme le polycaprolactone de formule
- $[(CH_2)_5\text{-}C(=O)\text{-}O\text{-}]_r\text{-}R_d$ avec r étant un entier allant de 10 à 500 et Rd représentant un groupe alkyle linéaire ou ramifié, comportant de 1 à 20 atomes de carbone, ou cyclique, comportant de 3 à 20 atomes de carbone ;

ou les polyesters aromatiques telles que le polyéthylènetéréphtalate et le polybutylènetéréphtalate de formules :

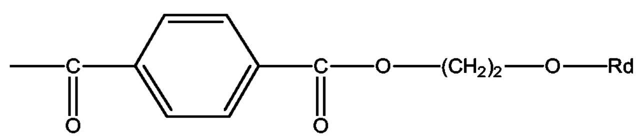

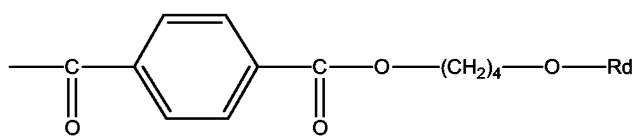

avec Rd répondant à la même définition que celle donnée précédemment.

Un autre mécanisme responsable de l'effet épaississant des dérivés de cellulose réside dans la création d'interactions hydrogène entre les groupements portés par R, lesdites interactions pouvant avoir lieu au sein d'une même molécule (« interactions intramoléculaires ») ou entre des molécules différentes (« interactions intermoléculaires »), ou de manière similaire dans la création d'interactions acide-base au sens de Lewis. Un tel mécanisme est le cas des groupes R porteurs de groupements aptes à établir des liaisons hydrogène et des groupes R polymères tels que polyamides, polyuréthanes et polyurées.

Enfin, un mécanisme responsable de l'effet épaississant des dérivés de cellulose peut résider en une organisation de type 'cristaux liquides' des groupes R greffés appropriés. Ceci est le cas, lorsque les groupes R représentent des groupes mésogènes, plus particulièrement des groupes mésogènes thermotropes de type smectique ou nématique, c'est-à-dire qu'ils vont se disposer sous formes de feuillets (« smectique ») ou de fils (« nématique »), qui vont ponter les molécules cellulosiques et piéger les solvants, en particulier, des esters de la phase organique de compositions de vernis à ongles dans un réseau tridimensionnel.

Des groupes R appropriés peuvent être des groupes de formule générale :

$$-E-F-E-G,$$

dans laquelle :

- les E, identiques ou différents, sont des groupes divalents aromatiques tels que :

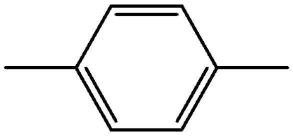

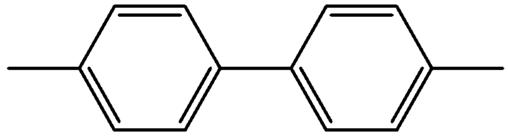

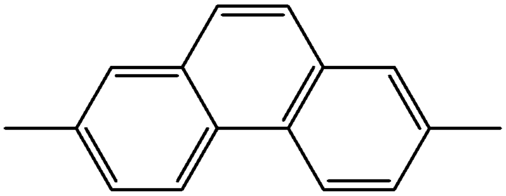

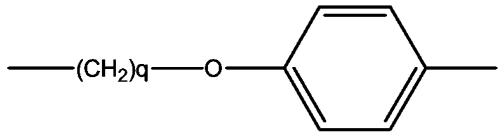

avec q représentant un entier allant de 1 à 10 ;

- F est un groupe divalent insaturé et non cyclique tel que :

-C=C-, -N=N-, -CH=N-, -CO-O-

ou une liaison simple;

- G représente un groupe terminal de formules suivantes:

- O-Ru, -CO-Ru, -CN, -COOH, $-(CH_2)_q$-Ru

avec Ru représentant un groupe alkyle linéaire ou ramifié comportant de 1 à 10 atomes de carbone et q représentant un entier allant de 1 à 10.

Des groupes particuliers répondant à cette définition sont les groupes de formules suivantes :

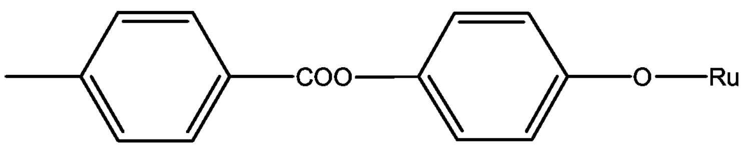

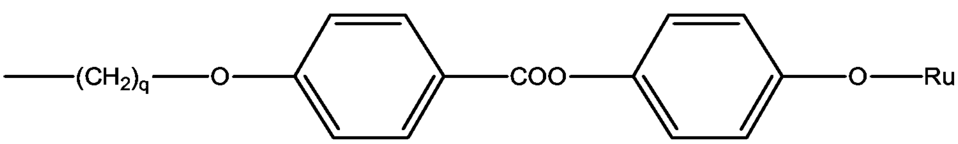

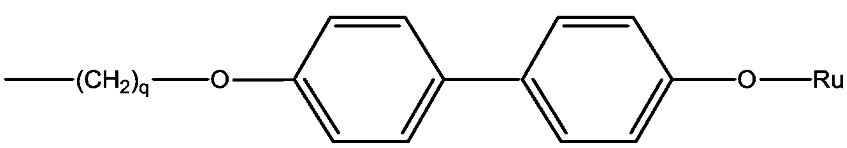

Des groupes R concernés par ce type de mécanisme peuvent être également des groupes dérivés de l'acide parahydroxybenzoïque répondant à l'une des formules suivantes :

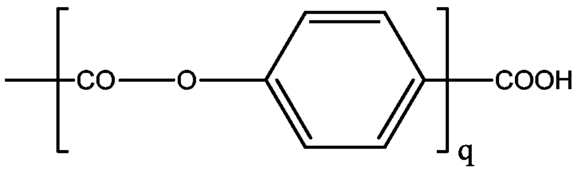

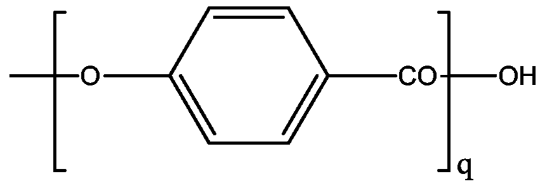

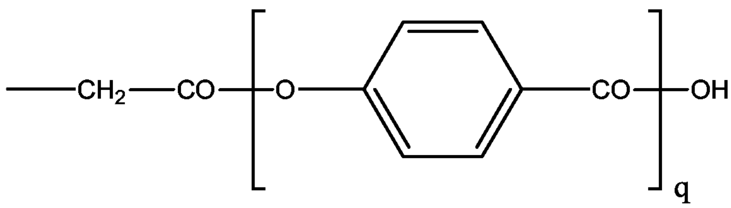

Des groupes R concernés par ce type de mécanisme peuvent être également des dérivés du cholestérol tel que le groupe de formule suivante :

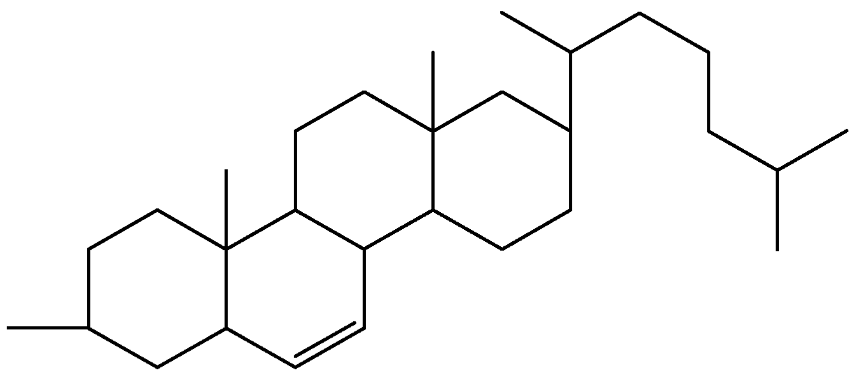

Enfin, des groupes R concernés par ce type de mécanisme peuvent des groupes répondant aux formules suivantes :

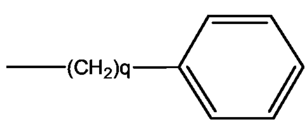

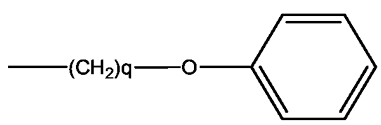

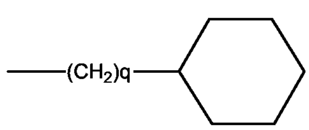

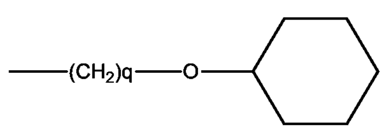

avec q étant un entier allant de 1 à 10.

Comme cela a été indiqué précédemment, les dérivés de cellulose modifiés décrits précédemment sont des agents épaississants de la phase organique de compositions de vernis à ongles, plus particulièrement lorsque celle-ci comprend des solvants esters. Ces dérivés peuvent également, en plus de leur fonction épaississante, assurer une fonction de filmogène dans les compositions de vernis à ongles dans lesquelles ils sont incorporés, étant entendu que les dérivés susmentionnés seront incorporés en plus grande quantité lorsqu'ils seront destinés à assurer en plus de la fonction épaississante une fonction filmogène.

De préférence, les compositions de l'invention sont des compositions aptes à former un film sans nécessiter de réticulation par voie thermique, chimique ou photochimique, et sont avantageusement exemptes d'agents de réticulation et d'amorceurs de réticulation, tels que des photoamorceurs. En d'autres termes, les compositions de l'invention sont aptes à former un film non réticulé par simple application de la composition sur un substrat suivie d'une évaporation du ou des solvants, ledit film étant éliminable par des dissolvants classiques dans le domaine des vernis à ongles.

Le ou les dérivés de cellulose modifiés conformes à l'invention peuvent représenter de 0,1 à 60%, de préférence de 0,5 à 40% et mieux encore de 1 à 30% en poids par rapport au poids total de la composition de vernis à ongles.

De préférence, lorsque que les dérivés de cellulose modifiés sont utilisés à la fois en tant qu'épaississants et en tant que filmogènes, la teneur de ces dérivés dans la composition de vernis à ongles va de 10 à 40 %, de préférence de 20 à 35% en poids par rapport au poids total de la composition.

De préférence, lorsque que les dérivés de cellulose modifiés sont utilisés uniquement en tant qu'épaississants, la teneur de ces dérivés dans la composition de vernis à ongles va de 0,1 à 20 %, de préférence de 1 à 10% en poids par rapport au poids total de la composition.

Les compositions de vernis à ongles, dans lesquelles les dérivés de cellulose décrits précédemment sont incorporés, peuvent être employées comme base pour vernis, comme produit de maquillage des ongles, comme composition de finition, encore appelée "top-coat" en terminologie anglo-saxonne, à appliquer sur le produit de maquillage des ongles ou bien encore comme produit de soin cosmétique des ongles. Ces compositions peuvent s'appliquer sur les ongles d'êtres humains ou bien encore sur des faux ongles.

Les compositions de vernis à ongles, dans lesquelles sont incorporés les dérivés de cellulose de l'invention, peuvent comprendre en plus de solvants esters, un ou plusieurs solvants choisis parmi :

- les cétones liquides à température ambiante tels que méthyléthylcétone, méthylisobutylcétone, diisobutylcétone, l'isophorone, la cyclohexanone, l'acétone ;
- les alcools liquides à température ambiante tels que l'éthanol, l'isopropanol, le diacétone alcool, le 2-butoxyéthanol, le cyclohexanol ;
- les glycols liquides à température ambiante tels que l'éthylène glycol, le propylène glycol, le pentylène glycol, le glycérol ;
- les éthers de propylène glycol liquides à température ambiante tels que le monométhyléther de propylène glycol, l'acétate de monométhyl éther de propylène glycol, le mono n-butyl éther de dipropylène glycol ;
- les esters à chaîne courte (ayant de 3 à 8 atomes de carbone au total) tels que l'acétate d'éthyle, l'acétate de méthyle, l'acétate de propyle, l'acétate de n-butyle, l'acétate d'isopentyle ;
- les éthers liquides à température ambiante tels que le diéthyléther, le diméthyléther ou le dichlorodiéthyléther ;
- les alcanes liquides à température ambiante tels que le décane, l'heptane, le dodécane, le cyclohexane ;
- les composés cycliques aromatiques liquides à température ambiante tels que le toluène et le xylène ;

et leurs mélanges.

La teneur en solvant(s) organique(s) (solvant ester et/ou solvant additionnel) de la composition de vernis à ongles peut aller de 20 à 90%, de préférence de 30 à 80% et mieux encore de 40 à 70% en poids par rapport au poids total de la composition.

La composition à phase organique peut comprendre en outre de l'eau, notamment en une teneur allant de 0,1 à 10% en poids, par rapport au poids total de la composition, de préférence, moins de 2 % d'eau en poids.

La composition peut comprendre également un ou plusieurs additifs choisis parmi les agents filmogènes, les agents plastifiants, les matières colorantes telles que les pigments, les nacres, les paillettes, des agents épaississants autres que les dérivés cellulosiques tels que décrits ci-dessus, les agents d'étalement, les agents mouillants, les agents dispersants, les anti-mousses, les conservateurs, les filtres UV, les actifs, les tensioactifs, les cires, les agents hydratants, les parfums, les neutralisants, les stabilisants, les antioxydants.

Ainsi, la composition peut comprendre un polymère filmogène additionnel qui peut être choisi parmi les nitrocelluloses, les esters de cellulose, les polymères radicalaires, les polycondensats et les polymères d'origine naturelle et leurs mélanges.

Le polymère filmogène additionnel peut être choisi notamment dans le groupe formé par les polymères vinyliques, les polyuréthannes, les polyesters, les résines alkydes, les résines époxyesters, les nitrocelluloses, les esters de cellulose, tels que les acétates, les propionates, les butyrates, les isobutyrates, les acétopropionates, les acétobutyrates de cellulose, les résines résultant de la condensation de formaldéhyde avec une arylsulfonamide, et leurs mélanges.

Le polymère filmogène additionnel peut être présent en une teneur allant de 0,1 % à 40 % en poids, par rapport au poids total de la composition, et de préférence allant de 1 % à 35 % en poids.

La composition peut comprendre, en outre, au moins un agent plastifiant. En particulier, on peut citer, seuls ou en mélange, les plastifiants usuels, tels que :

- les glycols et leurs dérivés tels que le diéthylène glycol éthyléther, le diéthylène glycol méthyléther, le diéthylène

glycol butyléther ou encore le diéthylène glycol hexyléther, l'éthylène glycol éthyléther, l'éthylène glycol butyléther, l'éthylène glycol hexyléther;

- les esters de glycérol,
- les dérivés de propylène glycol et en particulier le propylène glycol phényléther, le propylène glycol diacétate, le dipropylène glycol butyléther, le tripropylène glycol butyléther, le propylène glycol méthyléther, le dipropylène glycol éthyléther, le tripropylène glycol méthyléther et le diéthylène glycol méthyléther, le propylène glycol butyléther,
- des esters d'acides notamment carboxyliques, tels que des citrates, des phtalates, des adipates, des carbonates, des tartrates, des phosphates, des sébaçates,
- des dérivés oxyéthylénés tels que les huiles oxyéthylénées, notamment les huiles végétales telles que l'huile de ricin;
- leurs mélanges.

La quantité de plastifiant peut être choisie par l'homme du métier sur base de ses connaissances générales, de manière à obtenir une composition ayant des propriétés cosmétiquement acceptables. La teneur en plastifiant peut par exemple aller de 0,1 % à 15 % en poids, par rapport au poids total de la composition, et de préférence de 0,5 % à 10 % en poids.

La composition peut comprendre une matière colorante qui peut être choisie parmi les composés pulvérulents et/ou les colorants solubles dans le milieu de la composition. La matière colorante peut être présente en une teneur allant de 0,001 % à 10 % en poids, par rapport au poids total de la composition. Les composés pulvérulents peuvent être choisis parmi les pigments et/ou les nacres et/ou les paillettes habituellement utilisés dans les vernis à ongles.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique, les pigments métalliques comme l'aluminium ou le bronze. Parmi les pigments organiques, on peut citer le noir de carbone, les pigments de type D & C, et les laques à base de carmin de cochenille, de baryum, strontium, calcium, aluminium, la guanine.

Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les paillettes peuvent être choisies parmi celles en résine acrylique, polyester, polyéthylène téréphtalate, en aluminium.

Les colorants sont par exemple le rouge Soudan, le DC Red 17, le DC Green 6, le β-carotène, l'huile de soja, le brun Soudan, le DC Yellow 11, le DC Violet 2, le DC orange 5, le jaune quinoléine.

La composition selon l'invention peut en outre comprendre tout additif connu de l'homme du métier comme étant susceptible d'être incorporé dans une telle composition, tels que les agents épaississants, les agents d'étalement, les agents mouillants, les agents dispersants, les anti-mousses, les conservateurs, les filtres UV, les actifs, les tensioactifs, les cires, les agents hydratants, les parfums, les neutralisants, les stabilisants, les antioxydants. Bien entendu, l'homme du métier veillera à choisir ce ou ces éventuels composés complémentaires, et/ou leur quantité, de manière telle que les propriétés avantageuses de la composition pour l'utilisation selon l'invention ne soient pas, ou substantiellement pas, altérées par l'adjonction envisagée.

Les dérivés de cellulose utilisés comme agents épaississants selon l'invention peuvent être des dérivés disponibles dans le commerce mais peuvent être également préparés par différents procédés à la portée de l'homme du métier et, en particulier selon les deux grandes voies de synthèse suivantes :

- soit on part de celluloses déjà modifiées par des groupes nitro ou des groupes esters, et l'on fait réagir sur ces celluloses ainsi modifiées des réactifs appropriés pour greffer sur les fonctions hydroxyles libres des groupes 'épaississants' de formule -Y-R telle que définie précédemment (nommé à la suite de cette description Voie A) ;
- soit on part de celluloses déjà modifiées par des groupes -O-Y-R tels que définis précédemment et l'on fait réagir sur ces celluloses ainsi modifiées des réactifs adéquats pour obtenir la nitration ou l'estérification d'au moins une partie des fonctions hydroxyles libres (nommé voie B).

La voie préférentielle de synthèse des dérivés cellulosiques de l'invention est la voie A. Seule cette voie fera l'objet d'une description détaillée dans la présente demande.

Selon cette voie de synthèse, les celluloses de départ peuvent être des nitrocelluloses ou des esters de celluloses comportant un certain nombre de fonctions OH libres sur lesquelles vont réagir des réactifs appropriés pour donner des groupes - O-Y-R.

Les nitrocelluloses de départ utilisées pour introduire les groupes R tels que définis ci-dessus peuvent être

des nitrocelluloses filmogènes utilisées habituellement dans les encres, peintures et formulations de vernis à ongles. Ces nitrocelluloses sont préparées industriellement par estérification de la cellulose par un mélange d'acide nitrique et d'acide sulfurique, ce dernier jouant le rôle de déshydratant et déplaçant l'équilibre d'estérification. Elles constituent ainsi des nitrates de cellulose, désignés communément sous l'appellation de nitrocellulose.

De préférence, les nitrocelluloses de départ présentent un pourcentage d'azote de 10 à 13,5% en poids (une cellulose intégralement nitrée ayant un pourcentage d'azote de 14,14 %), ce qui correspond sensiblement à un taux de nitration de 1,7 à 2,5 groupes hydroxyles estérifiés par cycle anhydroglucose sur les 3 groupes -OH du cycle initialement disponibles pour la nitration. En d'autres termes, il reste entre 1 et 0,75 groupe -OH libre, pour le greffage de groupes R, par cycle anhydroglucose de la nitrocellulose.

En ce qui concerne le poids moléculaire des nitrocelluloses de départ, celui-ci est exprimé généralement par la mesure de la viscosité (par chute de bille) d'une solution du polymère (ou collodion) dans un mélange de solvants à un % de polymère donné, ledit pourcentage de polymère étant généralement de 12 à 25 %. En général, le mélange de solvants utilisé pour la caractérisation est constitué de 25 % d'éthanol à 95 % dénaturé, 20 % d'acétate d'éthyle et 55 % de toluène, les pourcentages étant exprimés en poids par rapport au mélange de solvants.

De préférence, les nitrocelluloses de départ, utilisées pour préparer les nitrocelluloses modifiées selon l'invention, ont des viscosités de chute de bille allant de (1/16) à 1000 secondes, de préférence de (1/4) à 200 secondes, pour une concentration en polymère de 12,2 % dans le mélange de solvants cité ci-dessus.

Du point de vue du conditionnement, les nitrocelluloses de départ, sont généralement, du fait de leur caractère inflammable et de leur pouvoir explosif, présentées :

- soit sous forme mouillée avec, par exemple, 35 % d'alcool (éthanol, isopropanol) ;
- soit en solution, dans un solvant dépourvu d'hydrogène labile;
- soit sous forme de 'chips', c'est-à-dire de mélange comprenant, par exemple, 80 % du polymère et 20 % d'un plastifiant, tel que le dibutylphtalate.

Compte tenu des dangers de manipulation de la nitrocellulose de départ, les procédés de préparation des nitrocelluloses modifiées selon l'invention s'effectueront, dans des conditions douces, à savoir :

- en opérant à une température de réaction de 0 à 80 °C, de préférence, de 20 à 60°C ;
- en opérant, de préférence, en l'absence d'oxygène, pour éviter tout risque d'explosion par contact de la nitrocellulose avec un oxydant, par exemple, en opérant sous un gaz inerte tel que l'argon.

Les esters de cellulose de départ utilisés pour greffer des chaînes hydrocarbonées R sont des dérivés de cellulose présentant un taux d'estérification suffisant pour conférer aux dérivés résultants une solubilité dans la phase organique de compositions de vernis à ongles, notamment lorsque celle-ci comprend un ou plusieurs solvants esters.

Que l'on travaille avec comme réactif de base des nitrocelluloses ou des esters de cellulose tels que définis précédemment, la réaction de greffage de groupes -Y-R tels que définis précédemment diffère selon que R est d'origine polymérique ou non.

Lorsque R est un groupe d'origine non polymérique (c'est-à-dire lorsque R répond aux définitions a), b), d) et e)), la réaction de greffage peut s'effectuer dans un solvant inerte par une réaction choisie parmi les réactions d'ethérification, estérification avec un acide carboxylique ou ses dérivés, transestérification avec un ester ou un carbonate, estérification avec un acide sulfonique ou ses dérivés, réaction avec un isocyanate, réaction avec un alcoxysilane.

Pour les parties qui suivent, dans l'exposé des méthodes de préparation des dérivés de cellulose (nitrocellulose ou ester de cellulose) modifiés selon l'invention, on utilisera les abréviations suivantes :

- Cell-OH pour le dérivé de cellulose de départ non modifié (nitrocellulose ou ester de cellulose), un seul OH, étant pris en compte, pour la clarté de l'exposé ;

R pour le groupe à greffer sur le dérivé de cellulose, répondant aux définitions données précédemment.

Dans la partie ci-après, on va décrire différentes réactions utilisables pour le greffage de chaînes R de nature non polymérique (c'est-à-dire répondant aux définitions a), b), d) et e).

1) Ethérification.

Pour l'éthérification, les réactions suivantes peuvent être envisagées, réactions pour lesquelles le radical Y faisant la jonction entre les chaînes R et la nitrocellulose est une simple liaison :

- réaction avec un halogénure d'alkyle R-X (X représentant un halogène), en milieu basique (par exemple, en présence d'une solution aqueuse d'hydroxyde de sodium) :

$$\text{Cell-OH} + \text{R-X} \rightarrow \text{Cell-OR} + \text{HX}$$

avec X étant un halogène choisi parmi le chlore, le brome ou l'iode.

- réaction avec un époxyde :

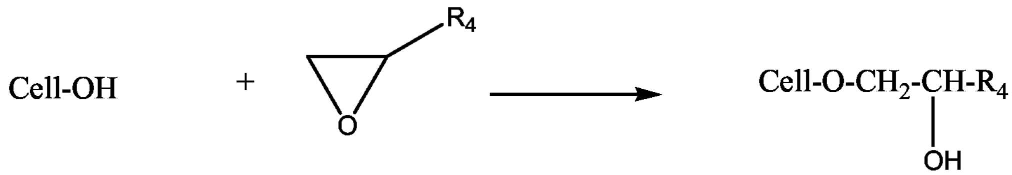

avec $R_4$ représentant une chaîne rentrant dans la constitution du groupe R défini précédemment, ledit groupe R étant représenté ici par le groupe $-CH_2-(CHOH)-R_4$; des réactifs époxydes appropriés peuvent être le 1,2-époxyoctane, le 1,2-époxynonane, le 1,2-époxydécane, le 1,2-époxynéodécane, le 1,2-époxycyclodécane, le 1,2-époxycyclododécane, le 1,2-époxycyclohexane, le 1,2-époxy-3-phénoxypropane.

- réaction avec un aldéhyde en milieu réducteur (tel que le triéthylsilane en présence de platine) :

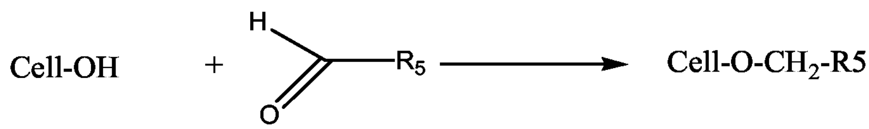

avec $R_5$ représentant une chaîne rentrant dans la constitution du groupe R, ledit groupe R étant représenté ici par le groupe $-CH_2-R_5$.

Selon une variante, cette réaction peut se dérouler en deux étapes, avec pour première étape une réaction préliminaire de l'aldéhyde avec un diol, tel que le glycol, pour former un acétal cyclique :

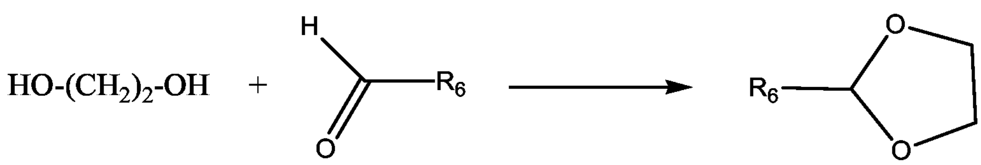

suivi d'une réaction de l'acétal cyclique avec la nitrocellulose :

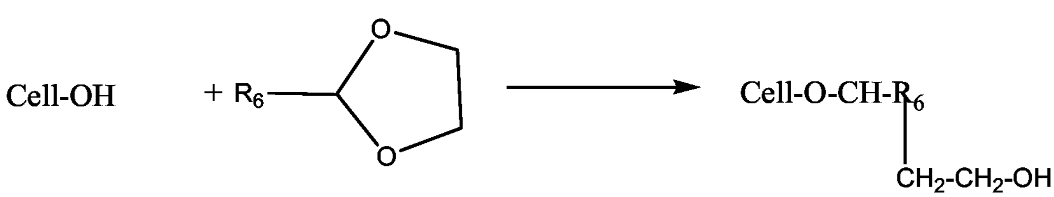

avec $R_6$ représentant une chaîne entrant dans la constitution du groupe R, ledit groupe R étant représenté ici par le groupe $-CH-(CH_2-CH_2-OH)-R_6$.

- réaction avec un éther mixte R-O-R' avec R' désignant un radical alkyle en $C_1$-$C_4$, en milieu acide :

$$\text{Cell-OH} + \text{R'-O-R} \rightarrow \text{Cell-O-R} + \text{R'-OH}$$

- réaction d'addition des -OH libres du dérivé de cellulose sur une double liaison, par exemple terminale, portée par le radical à greffer, en présence de $PdCl_2$ et $HgCl_2$ :

$$\textbf{Cell-OH + CH}_2\textbf{=CH-R}_7 \rightarrow \textbf{Cell-O-CH}_2\textbf{-CH}_2\textbf{-R}_7$$

ou

$$\textbf{Cell-OH + R}_a\textbf{-CH=CH-Rb} \rightarrow \textbf{Cell-O-CHR}_a\textbf{-CH}_2\textbf{-R}_b$$

$R_7$ représentant une chaîne entrant dans la constitution du groupe R, représenté ici par le groupe $-CH_2-CH_2-R_7$ et $R_a$ et $R_f$ représentant une chaîne entrant dans la constitution du groupe R, représenté ici par le groupe $-CHR_a-CH_2-R_b$.

D'autres réactions d'éthérification peuvent être envisagées, notamment celles mentionnées dans l'ouvrage « Advanced in Organic Chemistry », J.March, John Wiley & Son, Edition 1992.

2) Estérification.

A titre d'exemples pour les réactions d'estérification, on peut citer les réactions suivantes, réactions pour lesquelles Y représente un groupe de liaison divalent -CO- :

- réaction avec un acide carboxylique $R-CO_2H$ :

$$\textbf{Cell-OH + R-CO}_2\textbf{H} \rightarrow \textbf{Cell-O-CO-R + H}_2\textbf{O}$$

- réaction avec un chlorure d'acide R-COCl ou transestérification par un ester R-COOR', telle que :

$$\textbf{Cell-OH + R-COCl} \rightarrow \textbf{Cell-O-CO-R + HCl}$$

$$\textbf{Cell-OH + R-COO-R'} \rightarrow \textbf{Cell-O-CO-R + R'-OH}$$

- réaction avec un anhydride d'acide, par exemple :

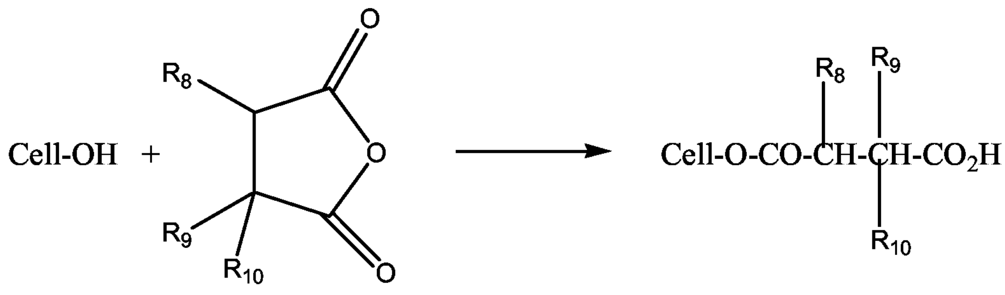

avec $R_8$, $R_9$ et $R_{10}$ étant tels que $-(CHR_8)-CR_9R_{10}-CO_2H$ représente R.

Des réactifs d'estérification appropriés peuvent être :

l'acide octanoïque, l'acide éthyl-2-hexanoïque, l'acide nonanoïque, l'acide décanoïque, l'acide néodécanoïque, l'acide undécanoïque, l'acide dodécanoïque, l'acide isononanoïque, l'acide palmitique, l'acide octadécanoïque, l'acide béhénique et leurs dérivés chlorures d'acide, anhydride d'acide.

3) Transestérification avec un carbonate.

La réaction suivante avec un carbonate R'-O-CO-O-R peut être envisagée, réaction pour laquelle Y représente un groupe de liaison divalent -CO-O-:

**Cell-OH + R'-O-CO-O-R → Cell-O-CO-O-R + R'-OH**

4) Estérification avec un chlorure de sulfonyle.

A titre d'exemples pour les réactions d'estérification avec un acide sulfonique ou un chlorure de sulfonyle, réactions pour lesquelles Y représente un groupe de liaison divalent -$SO_2$-, la réaction suivante peut être envisagée :

**Cell-OH + Cl-$SO_2$-R → Cell-O-$SO_2$-R + HCl**

5) Réaction avec un isocyanate.

A titre d'exemples pour les réactions de formation de liaisons carbamates, réactions pour lesquelles Y représente un groupe de liaison divalent - CO-NH-, la réaction suivante avec un isocyanate OCN-R peut être envisagée :

**Cell-OH + OCN-R → Cell-O-CO-NHR**

Des réactifs isocyanates appropriés peuvent être l'isocyanate de butyle, d'isobutyle, de pentyle, d'hexyle, d'heptyle, d'octyle, de 2-éthylhexyle, de nonyle, de décyle, d'undécyle, de dodécyle, de phényle.

6) Réaction avec un alcoxysilane.

A titre d'exemples pour les réactions avec un alcoxysilane, réactions pour lesquelles Y représente un groupe de liaison divalent -Si($R_{12}$)$_2$-, la réaction suivante peut être envisagée :

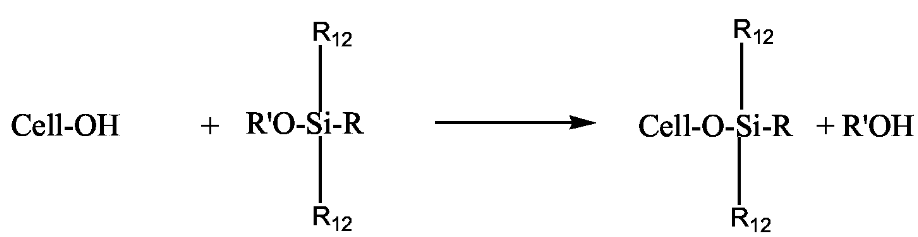

avec les $R_{12}$ identiques ou différents, pouvant être un groupe hydrocarboné, linéaire ou ramifié comportant de 1 à 500 atomes de carbone, ou cyclique comportant de 3 à 500 atomes de carbone, ledit groupe étant saturée ou insaturée et pouvant comporter un ou plusieurs hétéroatomes choisis parmi O, N, S, Si et/ou P, les $R_{12}$ ayant donc la même définition que les $R_3$ définis précédemment.

Lorsque R est un groupe d'origine polymérique, la réaction de greffage peut s'effectuer selon le schéma suivant :

Cell-OH + $X_1$-POL → Cell-O-Y-POL

avec POL représentant le polymère dont la séquence répond à la définition du groupe R donnée précédemment (dé-

finition c)), $X_1$ représentant une fonction portée par le polymère, ladite fonction étant réactive vis-à-vis des hydroxyles de dérivé cellulosique initiale (nitrocellulose ou ester de cellulose) et Y répondant à la même définition que celle donnée précédemment et résultant de la réaction de -OH avec $X_1$.

Au même titre que pour le greffage de chaînes hydrocarbonées de nature non polymérique, les fonctions réactives $X_1$ vis-à-vis des fonctions hydroxyles libres du dérivé cellulosique initial, peuvent être choisies parmi les fonctions époxyde, aldéhyde, acétal, halogène (chlore, brome, iode), éthylénique, acide carboxylique ou dérivé (chlorure, anhydride, ester d'alkyle en $C_1$-$C_4$), carbonate, acide sulfonique ou chlorure de sulfonyle, isocyanate, monoalcoxysilane.

Toutefois, compte tenu des dangers de la manipulation de la nitrocellulose mentionnée ci-dessus, les fonctions réactives $X_1$ des polymères hydrocarbonées, engagées dans la réaction de greffage, sont de préférence, choisies parmi les fonctions chlorure d'acide carboxylique, anhydride d'acide carboxylique, acide carboxylique (en présence d'un réactif de couplage du type DCCI), monoisocyanate, monoalcoxysilane, monoépoxyde, halogène (Cl, Br, I), monoéther d'alkyle $C_1$-$C_4$, double liaison vinylique.

Les polymères POL-$X_1$ de départ nécessitent d'être synthétisés, mis à part ceux pour lesquels $X_1$ est une double liaison réactive type vinylique, dont de nombreux sont disponibles dans le commerce. Les polymères POL-$X_1$ peuvent être synthétisés par exemple à partir d'un polymère comportant une fonction réactive différente de $X_1$ que l'on transforme par des réactions classiques en $X_1$ appropriée.

A titre d'exemples, on peut citer la réaction suivante :

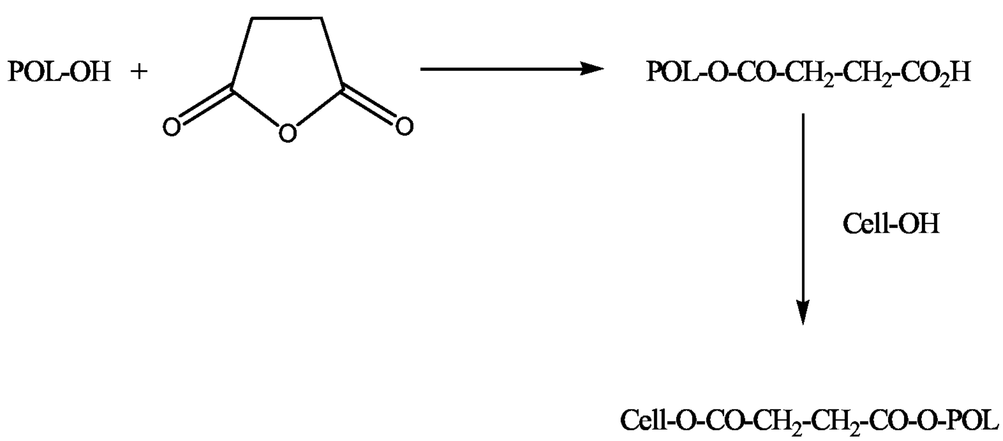


Selon une deuxième réalisation, la réaction de greffage peut consister, dans un premier temps, à transformer tout ou partie des fonctions hydroxyles du dérivé cellulosique de départ (nitrocellulose ou ester de cellulose) en fonctions réactives, puis, dans un deuxième temps, à faire réagir lesdites fonctions réactives avec les extrémités réactives adéquates de polymères comportant ladite chaîne hydrocarbonée R.

A titre d'exemples, on peut citer la réaction suivante :

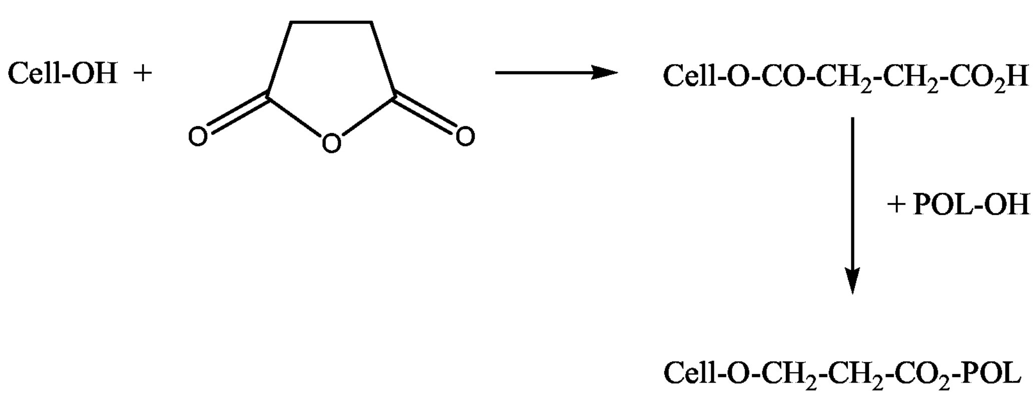


La réaction Cell-$OCOCH_2CH_2$-$CO_2H$ avec Cell-OH peut avoir lieu mais on se met dans des conditions, où

cette réaction est minimisée (on évite la réticulation).

Selon l'invention, les greffons polymères peuvent être :

- des polyoléfines (homo- ou copolymères), de préférence, semi-cristallins ;
- des polydiènes, de préférence hydrogénés ;
- des polycondensats tels que :

- des polyesters semi-cristallins aliphatiques comme la polycaprolactone ou aromatiques comme le polytéréphtalate d'éthylène glycol, le polytéréphtalate de butylène glycol ;
- des polyamides ;
- des polyuréthannes, des polyurées, des copolymères (urée/uréthanne) ;
- des polyorganosiloxanes.

A titre d'exemples de polyoléfines, on peut citer les polymères obtenus par homopolymérisation ou copolymérisation des monomères choisis parmi :

- les $\alpha$-oléfines, par exemple en $C_2$ à $C_{20}$, en particulier les copolymères d'$\alpha$-oléfines, dont les monomères donnent des homopolymères cristallins ; et des homopolymères ou copolymères d'$\alpha$-oléfines ramifiées. Citons en particulier l'homopolymère d'isobutylène et les copolymères entre éthylène (ou propylène) et d'$\alpha$-oléfines plus longues telles que le butène, l'hexène, l'octène, le décène, le dodécène. Pour ces $\alpha$-oléfines, on peut également citer leurs copolymères non cristallins avec les cyclooléfines, en particulier, les copolymères entre éthylène (ou propylène) et norbornène ou les dérivés du norbornène ;
- les diènes, par exemple, en $C_4$ à $C_{20}$, tels que le butadiène, l'isoprène, l'hexadiène, ...etc donnant des copolymères avec d'autres monomères vinyliques tel que les $\alpha$-oléfines mentionnées ci-dessus, et en plus avec le styrène ou styrènes substitués.

Comme indiqué précédemment, les chaînes polyoléfines ou polydiènes peuvent porter des groupes pouvant établir des interactions hydrogènes, en particulier, si les greffons polyoléfines ne sont pas cristallisables. Ces groupes pouvant établir des liaisons hydrogènes sont introduits dans les greffons par copolymérisation avec des monomères adéquats en particulier avec des monomères porteurs de groupes $-CO_2H$ tels que l'acide (méth)acrylique, l'acide crotonique, l'anhydride ou l'acide maléique, l'anhydride ou l'acide itaconique.

A titre d'exemples de polyesters semicristallins, on peut citer les polyesters semicristallins tels que ceux issus de la polyestérification entre les diols et diacides tels que l'éthylène glycol, le propylène glycol, le diéthylène glycol, le néopentyl glycol, le 1,4-butanediol, le furanne diméthanol, le cyclohexane diméthanol, le glycérol, le triméthylolpropane, le pentaérythitol et leurs mélanges, avec des acides polycarboxyliques, en particulier des acides carboxyliques et leurs dérivés esters en $C_1$-$C_4$, par exemple, l'acide succinique, glutarique et adipique ou leurs esters de diméthyle, l'anhydride phtalique ou le téréphtalate de diméthyle ou avec des lactones, par exemple la caprolactone ; ou avec des dimères cycliques tels que les polylactides ou les polyglycolides ;

- les polyesteramides obtenus par inclusion d'aminoalcools tels que l'éthanolamine dans des mélanges de polyestérification.

A titre d'exemples de polyamides, on peut citer les polyamides obtenus par condensation entre un diacide carboxylique (ou dérivé ester en $C_1$-$C_4$) aliphatique, cycloaliphatique ou aromatique en $C_3$-$C_{50}$ et une diamine en $C_2$-$C_{50}$ aliphatique, linéaire ou ramifiée, cycloaliphatique ou aromatique, les diacides pouvant être choisis parmi les diacides mentionnés ci-dessus, avec en plus les acides gras dimères (provenant de la condensation entre deux molécules de mono acides gras insaturés), les diamines pouvant être choisis parmi l'éthylènediamine, le 1,2-diaminopropane, le 1,3-diaminopropane, le 1,4-diaminobutane, le 1,2-diamino-2-méthylpropane, le 1,6-diaminohexane, le 1,10-diaminodécane,l'isophoronediamine, l'adamantanediamine, la 2,6-diaminopyridine ;

A titre d'exemples de polyuréthannes et de polyurées, on peut citer les polyuréthannes, polyurées et polyurées-uréthannes obtenus par polyaddition entre des diisocyanates aliphatiques, cycloaliphatiques et/ou aromatiques de $C_4$-$C_{100,}$ de préférence $C_4$-$C_{30}$ tels que l'hexaméthylène diisocyanate, l'isophorone diisocyanate, le toluène diisocyanate, le diphénylméthane diisocyanate et des diols tels que définis ci-dessus, ou des diamines telles que définies ci-dessus, ou des mélanges diols/diamines.

En ce qui concerne les polycondensats à extrémité réactive $X_1$, leur préparation, notamment en ce qui concerne les polyesters et les polyamides, ne nécessite pas d'aménagement particulier pour l'introduction du groupe réactif, dans la mesure où celui-ci existe déjà en extrémité de chaîne.

Par exemple, un polyester comporte, en général, en fin de préparation une extrémité $-CO_2H$ réactive et une

extrémité -OH. Il est à noter que cette extrémité -OH sera, de préférence, bloquée par un groupe inerte dépourvu d'hydrogène labile, afin de ne pas gêner la réaction de greffage sur le dérivé cellulosique de départ (nitrocellulose ou ester de cellulose) .

Les mêmes remarques sont applicables pour un polyamide, qui présente une extrémité $-CO_2H$ réactive et une extrémité $-NH_2$ à protéger par un groupe inerte vis-à-vis de la réaction de greffage avec le dérivé cellulosique de départ.

On peut introduire, également un groupe réactif $X_1$ au niveau du polycondensat, par introduction dans le milieu réactionnel, en cours de polycondensation, d'un réactif porteur du groupe $X_1$, qui doit être inerte vis-à-vis du type de polycondensation choisi ou inerte dans les conditions expérimentales de la polycondensation, et d'un seul groupe apte à participer à la polycondensation. Ce réactif est donc monofonctionnel vis-à-vis de la polycondensation et sert donc de limiteur de chaîne.

Ce réactif monofonctionnel vis-à-vis de la polycondensation et porteur d'un groupe réactif vis-à-vis des fonctions hydroxyles du dérivé cellulosique de départ, est, de préférence, introduit au cours de la polycondensation, pour que les chaînes du polymère ne soient terminées que par un seul groupe réactif $X_1$.

En ce qui concerne le greffage de chaînes polyorganosiloxanes, on peut utiliser un polymère à extrémité réactive, dont le squelette est un polyorganosiloxane. De tels polymères peuvent être des polymères commerciaux tels que les polydiméthylsiloxanes commercialisés par la société Shin-Etsu, lesdits polymères comprenant un enchaînement de motifs :

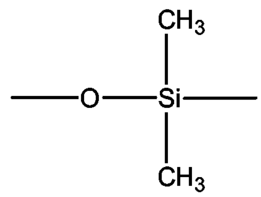

et une seule extrémité réactive du type -OH, époxyde ou éthylénique.

L'invention va maintenant être décrite en référence aux exemples suivants donnés à titre illustratif et non limitatif.

## DESCRIPTION DETAILLEE DE MODES DE REALISATION PARTICULIERS

Les exemples suivants illustrent la préparation de dérivés de cellulose, selon la présente invention, ainsi que des exemples de formulation, comprenant de tels dérivés de cellulose.

<u>EXEMPLE 1.</u>

Cet exemple expose la préparation d'un acétobutyrate de cellulose à groupes latéraux carbamate d'octadécyle.

Les réactifs utilisés sont les suivants :

- 100 g d'acétobutyrate de cellulose CAB 553-0,4 d'EASTMAN comportant 4,8 % en poids d'hydroxyles libres ;
- 10,4 g de monoisocyanate d'octadécyle ;
- 1000 g d'acétate d'éthyle ;
- 1g de 2-éthylhexanoate d'étain.

Le mode opératoire est le suivant :

Dans un réacteur muni d'une agitation centrale, d'un thermomètre, d'un réfrigérant, d'un système de barbotage d'azote et d'une ampoule à addition, on verse 900 g d'acétate d'éthyle puis, par petites portions, 100 g d'acétobutyrate de cellulose CAB 553-0,4 d'EASTMAN. On ajoute ensuite sous agitation et sous ébullition de l'acétate d'éthyle puis on ramène le milieu réactionnel à une température de 25°C, une fois que tout l'acétobutyrate de cellulose est dissout. On introduit alors dans l'ampoule l'isocyanate d'octadécyle préalablement dissous dans 100 g d'acétate d'éthyle et on ajoute le catalyseur 2-éthylhexanoate d'étain à la solution de polymère. Ensuite, en maintenant l'agitation avec barbotage d'azote, à température ambiante, la solution d'isocyante est ajoutée goutte à goutte dans le réacteur. On chauffe ensuite le mélange à 55°C sous agitation et barbotage d'azote. On maintient ces conditions pendant 8 heures.

On ramène ensuite la solution obtenue à température ambiante. On purifie par précipitation de la solution dans 10 mL d'heptane. On sèche le précipité formé sous vide. On obtient ainsi 105g d'acétobutyrate de cellulose à

groupes latéraux carbamate d'octadécyle.

EXEMPLE 2.

On prépare un vernis à ongles colorés ayant la composition suivante :

- Dérivé de cellulose de l'exemple 1: 3 g
- Plastifiant: 7 g
- Alcool isopropylique: 5 g
- Polymères filmogènes: 28 g
- Pigments: 1 g
- Acétate d'éthyle/acétate de butyle: qsp 100 g

Les polymères filmogènes sont constitués d'un mélange de nitrocellulose et de co-résines.

Après application de la composition de vernis à ongles et après séchage, on obtient un film lisse, homogène et brillant.

## Revendications

1. Utilisation en tant qu'agent épaississant de la phase organique d'une composition de vernis à ongles de dérivés cellulosiques choisis parmi les nitrocelluloses, les esters de cellulose, lesdits dérivés comportant des fonctions hydroxyles libres remplacées, en tout ou partie, par des radicaux de formule -OYR, dans laquelle :

   - R représente un groupe choisi parmi :

     a) les groupes hydrocarbonés à chaînes linéaires ou ramifiées, saturés ou insaturés, ou cycliques, saturés ou insaturés,
     lesdits groupes pouvant comporter dans leurs chaînes un ou plusieurs groupes aromatiques et/ou un ou plusieurs hétéroatomes choisis parmi O, N, P, Si, S ;
     b) les groupes fluoro- ou perfluoroalkyles ;
     c) les groupes de nature polymérique choisis parmi les polyoléfines, les polydiènes, les polycondensats ;
     d) les groupes organosiloxanes ou polyorganosiloxanes ;
     e) les groupes mésogènes

     lesdits groupes répondant à la définition a), b), c), d) ou e) pouvant comporter au moins un groupement apte à établir une liaison hydrogène,
   - Y représente une liaison simple ou un groupe de liaison divalent.

2. Utilisation selon la revendication 1, dans laquelle la phase organique de la composition de vernis à ongles comprend au moins un solvant ester.

3. Utilisation selon la revendication 1 ou 2, dans laquelle le groupe de liaison divalent Y est choisi parmi les groupes -(C=O)-, -(C=O)O-, $-SO_2-$, -CO-NH- ou -CO-NR' -, $-Si(R_3)_2-$, les $R_3$ identiques ou différents, étant un groupe hydrocarboné linéaire ou ramifiée, comportant de 1 à 500 atomes de carbone, ou cyclique comportant de 3 à 500 atomes de carbone, ledit groupe étant saturé ou insaturé et pouvant comporter un ou plusieurs atomes de O, N, S, Si et/ou P et R' désignant un radical alkyle comportant de 1 à 4 atomes de carbone.

4. Utilisation selon l'une quelconque des revendications 1 à 3, dans laquelle le groupe R, lorsqu'il répond à la définition a), est un groupe alkyle linéaire ou ramifié comportant de 1 à 50 atomes de carbone, de préférence de 8 à 50 atomes de carbone.

5. Utilisation selon l'une quelconque des revendications 1 à 3, dans laquelle le groupe R, lorsqu'il répond à la définition b), est un groupe fluoro- ou perfluoroalkyle comportant de 6 à 50 atomes de carbone.

6. Utilisation selon l'une quelconque des revendications 1 à 3, dans laquelle le groupe R, lorsqu'il représente une polyoléfine selon la définition c), est une polyoléfine choisie parmi les polyéthylènes, les copolymères (éthylène/propylène), les copolymères (éthylène/butène), les copolymères (éthylène/héxène), les copolymères (éthylène/octène).

**7.** Utilisation selon l'une quelconque des revendications 1 à 3, dans laquelle le groupe R, lorsqu'il représente un polycondensat selon la définition c), est choisi parmi des polyesters, des polyamides, des polyesteramides, des polyuréthannes, des polycarbonates, des polyurées, des copolymères (urée/uréthanne), des polyéthers.

**8.** Utilisation selon l'une quelconque des revendications 1 à 3, dans laquelle, lorsque R est un groupe répondant à la définition d), il répond à la formule suivante :

$$-[Si(Ra,Rb)-O-]_p-Si(Ra, Rb, Rc)$$

dans laquelle :

les Ra, Rb et Rc, identiques ou différents représentent une chaîne alkyle linéaire ou ramifiée, comportant de 1 à 20 atomes de carbone, un phényle ou une chaîne perfluoroalkyle comportant de 3 à 20 atomes de carbone et p étant un entier allant de 0 à 500.

**9.** Utilisation selon l'une quelconque des revendications 1 à 3, dans laquelle, lorsque R est un groupe répondant à la définition e), il est choisi parmi les groupes alkylaryles, alkylcycloalkyles, alcoxyaryles, alcoxycycloalkyles.

**10.** Utilisation selon la revendication 9, dans laquelle R est choisi parmi les groupes de formules suivantes :

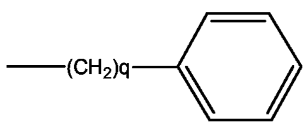
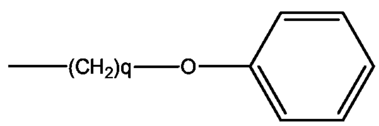

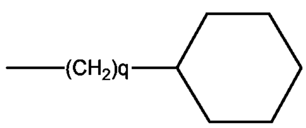
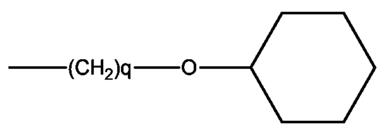

avec q étant un entier allant de 1 à 10.

**11.** Utilisation selon l'une quelconque des revendications 1 à 3, dans laquelle, lorsque R est un groupe répondant à la définition e), il est représenté par la formule suivante :

$$-E-F-E-G,$$

dans laquelle :

- les E, identiques ou différents, sont des groupes divalents aromatiques ;
- F est un groupe divalent insaturé et non cyclique ou une liaison simple ;
- G représente un groupe terminal de formules suivantes:

$$-O-Ru,\ -CO-Ru,\ -CN,\ -COOH,\ -(CH_2)_q-Ru$$

avec Ru représentant un groupe alkyle linéaire ou ramifié comportant de 1 à 10 atomes de carbone et q répondant à la définition donnée à la revendication 10.

**12.** Utilisation selon la revendication 11, dans laquelle R est choisi parmi les groupes de formules suivantes :

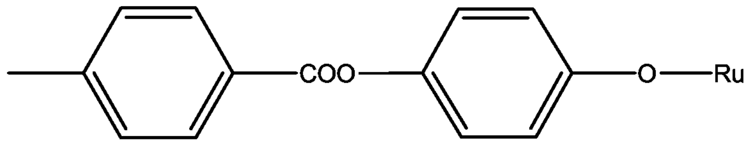

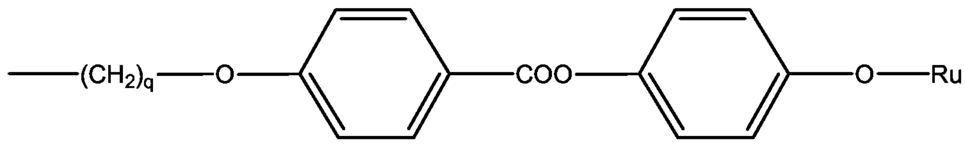

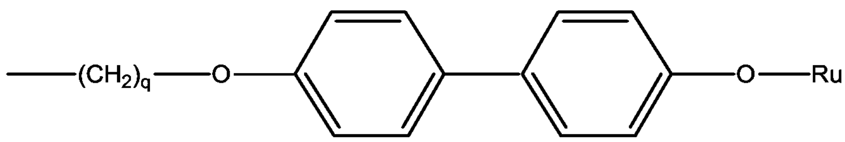

avec Ru répondant à la définition de la revendication 11 et q répondant à la définition de la revendication 10.

**13.** Utilisation selon l'une quelconque des revendications 1 à 3, dans laquelle, lorsque R est un groupe répondant à la définition e), il représente un groupe dérivé de l'acide parahydroxybenzoïque répondant à l'une des formules suivantes :

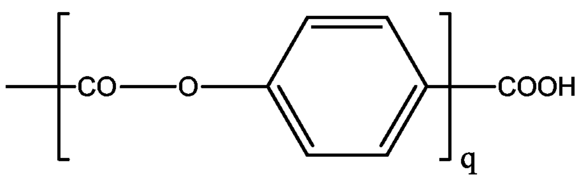

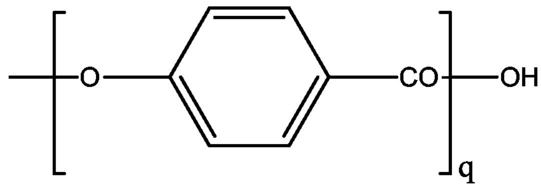

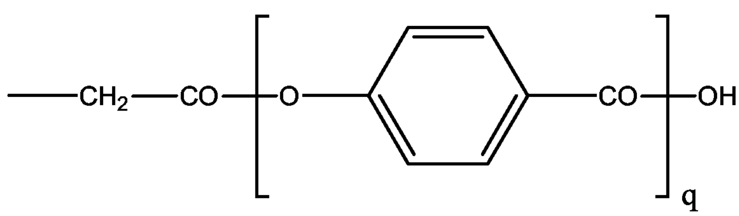

avec q répondant à la définition donnée à la revendication 10.

**14.** Utilisation selon l'une quelconque des revendications 1 à 13, dans laquelle le groupement apte à établir une liaison hydrogène est choisi parmi les groupes de formules suivantes :

- hydroxyle -OH ;
- acide carboxylique -COOH ;
- amino $-NR_1R_2$ avec $R_1$ et $R_2$, identiques ou différents ;
- pyridino de formule :

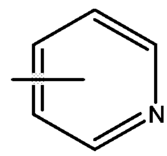

- pyrimidino de formule :

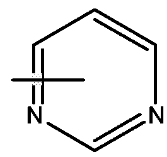

- oxazolino répondant à l'une des formules suivantes :

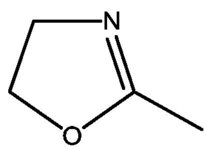
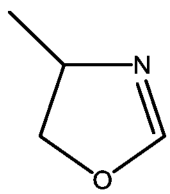
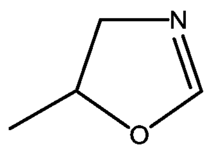

- amido de formules -NH-CO-R' ou -CO-NH-$R_1$;
- pyrrolidono répondant à l'une des formules suivantes :

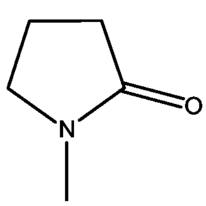
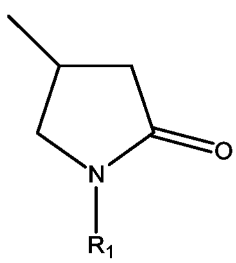
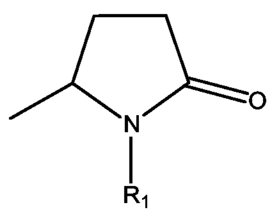
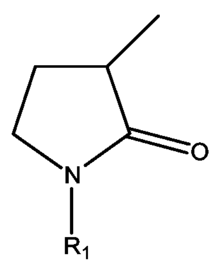

- carbamoyl de formules -O-CO-NH-R' ou -NH-CO-O-R' ;
- thiocarbamoyl de formules -O-CS-$NHR_1$ ou -NH-CS-O-R' ;
- carbonato -O-CO-O-R';
- uréyl-$NR_1$-CO-N $(R_1)_2$, les $R_1$ étant identiques ou différents ;
- thiouréyl -$NR_1$-CS-N$(R_1)_2$, les $R_1$ étant identiques ou différents ;
- oxamido -$NR_1$-CO-CO-N$(R_1)_2$ avec les $R_1$ identiques ou différents ;
- guanidino -NH-C(=NH)-N$(R_1)_2$ avec les $R_1$ identiques ou différents ;
- biguanidino -NH-C(=NH)-NH-C(=NH)-N$(R_1)_2$ avec les $R_1$ identiques ou différents ;
- sulfonamido -$NR_1$-S$(=O)_2$-R',

avec $R_1$ et $R_2$ représentant H ou un groupe alkyle comprenant de 1 à 4 atomes de carbone, R' représentant un radical alkyle comprenant de 1 à 4 atomes de carbone.

**15.** Composition de vernis à ongles comprenant une phase organique à base d'au moins un solvant organique et comprenant au moins un dérivé cellulosique comprenant des fonctions hydroxyles remplacées en tout ou partie par des radicaux de formule -OYR, dans laquelle :

- R représente un groupe choisi parmi :

  a) les groupes hydrocarbonés à chaînes linéaires ou ramifiées, saturés ou insaturés, ou cycliques, saturés ou insaturés,
  lesdits groupes pouvant comporter dans leurs chaînes un ou plusieurs groupes aromatiques et/ou un ou plusieurs hétéroatomes choisis parmi O, N, P, Si, S ;
  b) les groupes fluoro- ou perfluoroalkyles ;
  c) les groupes de nature polymérique choisis parmi les polyoléfines, les polydiènes, les polycondensats ;
  d) les groupes organosiloxanes ou polyorganosiloxanes ;
  e) les groupes mésogènes;

  lesdits groupes répondant à la définition a), b), c), d) ou e) pouvant comporter au moins un groupement apte à établir une liaison hydrogène,
- Y représente une liaison simple ou un groupe de liaison divalent,

ledit dérivé cellulosique étant un ester de cellulose lorsque R répond à la définition a), c) et d), un ester de cellulose ou une nitrocellulose lorsque R répond à la définition b), e).

**16.** Composition selon la revendication 15, dans laquelle le ou les dérivés de cellulose représente(nt) de 0,1 à 60%, de préférence de 0,5 à 40% et mieux encore de 1 à 30% en poids par rapport au poids total de la composition de vernis à ongles.

**17.** Composition selon la revendication 15 ou la revendication 16, dans laquelle la phase organique de la composition de vernis à ongles comprend au moins un solvant ester.

**18.** Composition selon la revendication 17, pour laquelle le solvant ester est un ester comprenant de 3 à 8 atomes de carbone.

**19.** Composition selon l'une quelconque des revendications 15 à 18, dans laquelle le ou les solvants organiques sont présents à une teneur de 20 à 90%, de préférence de 30 à 80% et mieux encore de 40 à 70% en poids par rapport au poids total de la composition.

**20.** Composition selon l'une quelconque des revendications 15 à 19, dans laquelle le groupe de liaison divalent Y est choisi parmi les groupes -(C=O)-, -(C=O)O- $-SO_2-$, -CO-NH- ou -CO-NR' -, $-Si(R_3)_2-$, les $R_3$ identiques ou différents, étant un groupe hydrocarboné linéaire ou ramifiée, comportant de 1 à 500 atomes de carbone, ou cyclique comportant de 3 à 500 atomes de carbone, ledit groupe étant saturé ou insaturé et pouvant comporter un ou plusieurs atomes de O, N, S, Si et/ou P et R' désignant un radical alkyle comportant de 1 à 4 atomes de carbone.

**21.** Composition selon l'une quelconque des revendications 15 à 20, dans laquelle le groupe R, lorsqu'il répond à la définition a), est un groupe alkyle linéaire ou ramifié comportant de 1 à 50 atomes de carbone, de préférence de 8 à 50 atomes de carbone.

**22.** Composition selon l'une quelconque des revendications 15 à 20, dans laquelle le groupe R, lorsqu'il répond à la définition b), est un groupe fluoro- ou perfluoroalkyle comportant de 6 à 50 atomes de carbone.

**23.** Composition selon l'une quelconque des revendications 15 à 20, dans laquelle le groupe R, lorsqu'il représente une polyoléfine selon la définition c), est une polyoléfine choisie parmi les polyéthylènes, les copolymères (éthylène/propylène), les copolymères (éthylène/butène), les copolymères (éthylène/héxène), les copolymères (éthylène/octène).

**24.** Composition selon l'une quelconque des revendications 15 à 20, dans laquelle le groupe R, lorsqu'il représente un polycondensat selon la définition c), est choisi parmi des polyesters, des polyamides, des polyesteramides, des polyuréthannes, des polycarbonates, des polyurées, des copolymères (urée/uréthanne), des polyéthers.

**25.** Composition selon l'une quelconque des revendications 15 à 20, dans laquelle, lorsque R est un groupe répondant à la définition d), il répond à la formule suivante :

$$- [Si(Ra,Rb)\text{-}O\text{-}]_p\text{-}Si\ (Ra, Rb, Rc)$$

dans laquelle :

les Ra, Rb et Rc, identiques ou différents représentent une chaîne alkyle linéaire ou ramifiée, comportant de 1 à 20 atomes de carbone, un phényle ou une chaîne perfluoroalkyle comportant de 3 à 20 atomes de carbone et p étant un entier allant de 0 à 500.

**26.** Composition selon l'une quelconque des revendications 15 à 20, dans laquelle, lorsque R est un groupe répondant à la définition e), il est choisi parmi les groupes alkylaryles, alkylcycloalkyles, alcoxyaryles, alcoxycycloalkyles.

**27.** Composition selon la revendication 26, dans laquelle R est choisi parmi les groupes de formules suivantes :

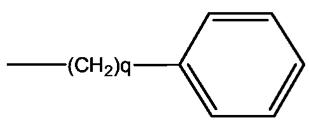
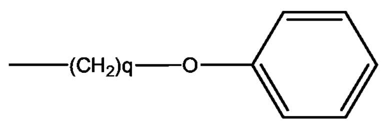
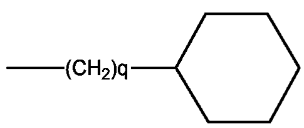
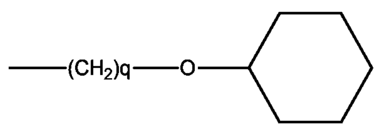

avec q étant un entier allant de 1 à 10.

**28.** Composition selon l'une quelconque des revendications 15 à 20, dans laquelle, lorsque R est un groupe répondant à la définition e), il est représenté par la formule suivante :

-E-F-E-G,

dans laquelle :

- les E, identiques ou différents, sont des groupes divalents aromatiques ;
- F est un groupe divalent insaturé et non cyclique ou une liaison simple ;
- G représente un groupe terminal de formules suivantes:

$$\text{-O-Ru, -CO-Ru, -CN, -COOH, -}(CH_2)_q\text{-Ru}$$

avec Ru représentant un groupe alkyle linéaire ou ramifié comportant de 1 à 10 atomes de carbone et q répondant à la définition donnée à la revendication 27.

**29.** Composition selon la revendication 28, dans laquelle R est choisi parmi les groupes de formules suivantes :

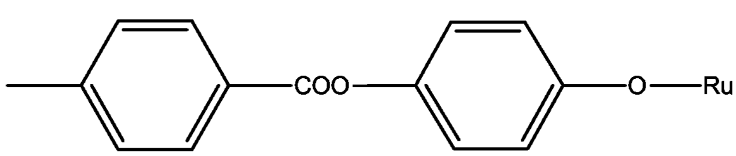

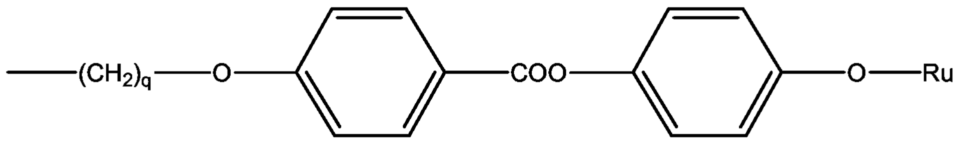

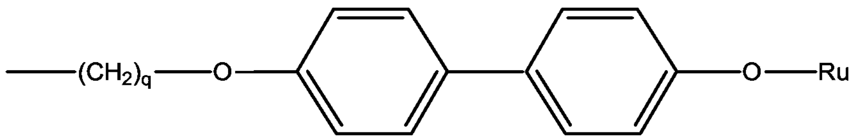

avec Ru répondant à la définition donnée à la revendication 28 et q répondant à la définition donnée à de la revendication 27.

**30.** Composition selon l'une quelconque des revendications 15 à 20, dans laquelle, lorsque R est un groupe répondant à la définition e), il représente un groupe dérivé de l'acide parahydroxybenzoïque répondant à l'une des formules suivantes :

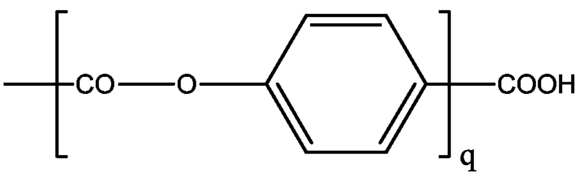

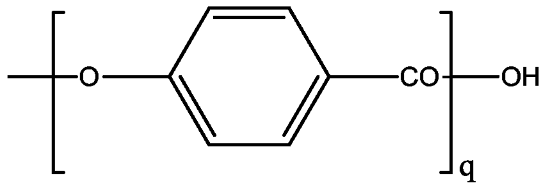

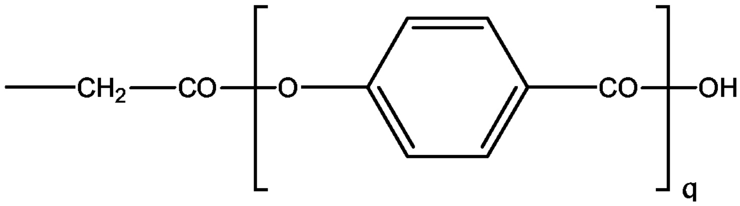

avec q répondant à la définition donnée à la revendication 27.

**31.** Composition selon l'une quelconque des revendications 15 à 30, dans laquelle le groupement apte à établir une liaison hydrogène est choisi parmi les groupes de formules suivantes :

- hydroxyle -OH ;
- acide carboxylique -COOH ;
- amino $-NR_1R_2$ avec $R_1$ et $R_2$, identiques ou différents ;
- pyridino de formule :

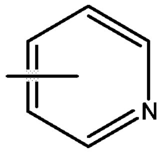

- pyrimidino de formule :

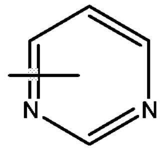

- oxazolino répondant à l'une des formules suivantes :

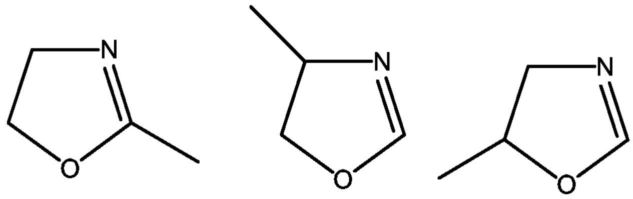

- amido de formules -NH-CO-R' ou -CO-NH-$R_1$;
- pyrrolidono répondant à l'une des formules suivantes :

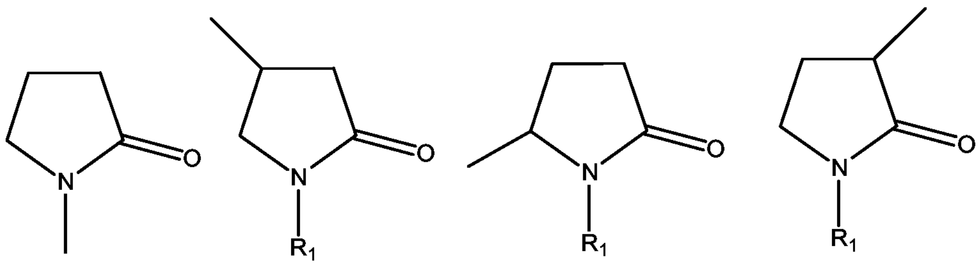

- carbamoyl de formules -O-CO-NH-R' ou -NH-CO-O-R' ;
- thiocarbamoyl de formules -O-CS-$NHR_1$ ou -NH-CS-O-R' ;
- carbonato -O-CO-O-R';
- uréyl-$NR_1$-CO-N ($R_1$) $_2$, les $R_1$ étant identiques ou différents ;
- thiouréyl -$NR_1$-CS-N$(R_1)_2$, les $R_1$ étant identiques ou différents ;
- oxamido -$NR_1$-CO-CO-N$(R_1)_2$ avec les $R_1$ identiques ou différents ;
- guanidino -NH-C(=NH)-N$(R_1)_2$ avec les $R_1$ identiques ou différents ;
- biguanidino -NH-C(=NH)-NH-C(=NH)-N$(R_1)_2$ avec les Ri identiques ou différents ;
- sulfonamido -$NR_1$-S$(=O)_2$-R',

avec $R_1$ et $R_2$ représentant H ou un groupe alkyle comprenant de 1 à 4 atomes de carbone, R' représentant un radical alkyle comprenant de 1 à 4 atomes de carbone.

**32.** Composition selon l'une quelconque des revendications 15 à 31, comprenant, en outre, un ou plusieurs additifs choisis parmi les agents filmogènes, les agents plastifiants, les matières colorantes telles que les pigments, les nacres, les paillettes, les agents épaississants autres que les dérivés cellulosiques tels que définis dans les revendications 15 à 31, les agents d'étalement, les agents mouillants, les agents dispersants, les anti-mousses, les conservateurs, les filtres UV, les actifs, les tensioactifs, les cires, les agents hydratants, les parfums, les neutralisants, les stabilisants, les antioxydants.

Office européen des brevets

**RAPPORT PARTIEL DE RECHERCHE EUROPEENNE**
qui selon la règle 45 de la Convention sur le brevet européen est consideré, aux fins de la procédure ultérieure, comme le rapport de la recherche européenne

Numéro de la demande
EP 04 10 3198

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X | HIROSE JUNICHI: "Aqueous enamels containing carboxyalkyl cellulose nitrates and polymethyacrylates for nails" CHEMABS, 2002, XP002225100<br>* abrégé *<br>& DATABASE WPI<br>Section Ch, Week 200124<br>Derwent Publications Ltd., London, GB;<br>Class A11, AN 2001-229524<br>& JP 2001 019892 A (ASAHI KASEI KOGYO KK)<br>1 mars 2001 (2001-03-01)<br>* abrégé *<br>----- | 1-3, 14-16, 19,20, 31,32 | A61K7/043<br>A61K7/04<br>C09D101/10<br>C09D101/18 |
| X | US 4 683 007 A (HOROWITZ CARL ET AL)<br>28 juillet 1987 (1987-07-28)<br>* colonne 1, ligne 34-48 *<br>* colonne 2, ligne 3 - colonne 3, ligne 38; revendications *<br>----- | 1,2,4,6, 15-19, 23,32 | |
| | -/-- | | |

| DOMAINES TECHNIQUES RECHERCHES (Int.Cl.7) |
|---|
| A61K<br>C09D |

## RECHERCHE INCOMPLETE

La division de la recherche estime que la présente demande de brevet, ou une ou plusieurs revendications, ne sont pas conformes aux dispositions de la CBE au point qu'une recherche significative sur l'état de la technique ne peut être effectuée, ou seulement partiellement, au regard de ces revendications.

Revendications ayant fait l'objet d'une recherche complète:

Revendications ayant fait l'objet d'une recherche incomplète:

Revendications n'ayant pas fait l'objet d'une recherche:

Raison pour la limitation de la recherche:

voir feuille supplémentaire C

| Lieu de la recherche | Date d'achèvement de la recherche | Examinateur |
|---|---|---|
| Munich | 29 septembre 2004 | Loloiu, C |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P04C08)

Office européen
des brevets

**RECHERCHE INCOMPLETE**
**FEUILLE SUPPLEMENTAIRE C**

Numéro de la demande
EP 04 10 3198

Revendications ayant fait l'objet de recherches complètes:
2-8,14, 16-25,31-32

Revendications ayant fait l'objet de recherches incomplètes:
1,9-13,15,26-30

Revendications n'ayant pas fait l'objet de recherches:
-

Raison pour la limitation de la recherche:

Les revendications 1,9-13,15,26-30 présentes ont trait à une très grande variété de composés. En fait, lesdites revendications contiennent tant d'options, de variables, de permutations possibles et de conditions que le manque de clarté (et/ou de concision) au sens de l'Article 84 CBE qui s'en suit, est d'une importance telle qu'une recherche significative de l'objet des revendications devient impossible. Un fondement au sens de l'Article 84 CBE et/ou un exposé au sens de l'Article 83 CBE ne peut cependant être trouvé que pour un nombre très restreint de ces composés revendiqués.
En particulier la definition des groupes "mésogènes" n'est pas sufissament claire pour pour permettre établir les limites de l'entendu des revendications les incluant.
Les revendications manquent à un tel point de fondement et l'exposé de l'invention dans la description est si limité q'une recherche significative couvrant tout le spectre revendiqué est impossible.
Par conséquent, la recherche a été effectuée pour les parties de la demande qui apparaissent être claires et/ou concises c'est à dire les composés definis dans les revendications independantes 1 et 15 en combinason avec les revendications dependantes 2-8,14,16-25,31-32.

Office européen des brevets

# RAPPORT PARTIEL DE RECHERCHE EUROPEENNE

Numero de la demande
EP 04 10 3198

| DOCUMENTS CONSIDERES COMME PERTINENTS | | | CLASSEMENT DE LA DEMANDE (Int.Cl.7) |
|---|---|---|---|
| Catégorie | Citation du document avec indication, en cas de besoin, des parties pertinentes | Revendication concernée | |
| X | US 5 985 951 A (COOK PHILLIP MICHAEL) 16 novembre 1999 (1999-11-16)<br>* colonne 2, ligne 11 - colonne 4, ligne 50 *<br>* colonne 5, ligne 11 - colonne 6, ligne 56 *<br>* colonne 10, ligne 30 - ligne 54; revendications; exemples 1-7 *<br>----- | 1-4, 14-21, 31,32 | |
| X | DATABASE CA [Online] CHEMICAL ABSTRACTS SERVICE, COLUMBUS, OHIO, US; 1 octobre 1994 (1994-10-01), UCHIDA, SATORU ET AL: "nail lacquers containing silicone cellulose derivatives" XP002274741 extrait de STN Database accession no. 121:163709<br>* abrégé *<br>& JP 06 157246 A (SHINETSU CHEMICAL INDUSTRY CO., LTD., JAPAN) 3 juin 1994 (1994-06-03)<br>----- | 1-3,8, 14-20, 25,31,32 | DOMAINES TECHNIQUES RECHERCHES (Int.Cl.7) |
| X | US 2001/041168 A1 (RAMIN ROLAND) 15 novembre 2001 (2001-11-15)<br>* page 1, alinéa 9 - page 2, alinéa 37; revendications; exemples 1-24 *<br>----- | 1-5, 14-21, 31,32 | |
| X | EP 1 095 959 A (SHINETSU CHEMICAL CO) 2 mai 2001 (2001-05-02)<br>* page 2, ligne 35 - page 3, ligne 49 *<br>* page 6, ligne 39 - page 7, ligne 33; exemple 6 *<br>----- | 1,3,8, 14-16, 19,20, 25,31,32 | |
| | -/-- | | |

EPO FORM 1503 03.82 (P04C11)

Office européen des brevets

# RAPPORT PARTIEL DE RECHERCHE EUROPEENNE

Numero de la demande
EP 04 10 3198

| Catégorie | Citation du document avec indication, en cas de besoin, des parties pertinentes | Revendication concernée | CLASSEMENT DE LA DEMANDE (Int.Cl.7) |
|---|---|---|---|
| A | GB 1 120 373 A (ICI LTD)<br>17 juillet 1968 (1968-07-17)<br>* page 1, ligne 12 - page 2, ligne 6; revendications; exemples 1-12 *<br>----- | 1,3,5,<br>14,15,22 | |
| P,X | EP 1 342 731 A (OREAL)<br>10 septembre 2003 (2003-09-10)<br><br>* page 2, ligne 5-9 - ligne 28-51 *<br>* page 6, ligne 51 - page 8, ligne 20 *<br>* page 5, ligne 18 - ligne 47 *<br>* page 19; revendications; exemples 1-4 *<br>* page 15, ligne 53 - page 17, ligne 8 *<br>----- | 1-4,6-8,<br>14-21,<br>23-25,<br>31,32 | |

DOMAINES TECHNIQUES RECHERCHES (Int.Cl.7)

EPO FORM 1503 03.82 (P04C11)

**ANNEXE AU RAPPORT DE RECHERCHE EUROPEENNE RELATIF A LA DEMANDE DE BREVET EUROPEEN NO.** EP 04 10 3198

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

29-09-2004

| Document brevet cité au rapport de recherche | | Date de publication | Membre(s) de la famille de brevet(s) | | | Date de publication |
|---|---|---|---|---|---|---|
| US 4683007 | A | 28-07-1987 | AUCUN | | | |
| US 5985951 | A | 16-11-1999 | WO | 9848769 | A1 | 05-11-1998 |
| JP 6157246 | A | 03-06-1994 | JP | 2953601 | B2 | 27-09-1999 |
| US 2001041168 | A1 | 15-11-2001 | FR | 2785533 | A1 | 12-05-2000 |
| | | | AT | 219920 | T | 15-07-2002 |
| | | | BR | 9907314 | A | 24-04-2001 |
| | | | CA | 2288246 | A1 | 06-05-2000 |
| | | | CN | 1260167 | A | 19-07-2000 |
| | | | DE | 69901993 | D1 | 08-08-2002 |
| | | | DE | 69901993 | T2 | 27-02-2003 |
| | | | EP | 1000609 | A1 | 17-05-2000 |
| | | | ES | 2180259 | T3 | 01-02-2003 |
| | | | JP | 2000143447 | A | 23-05-2000 |
| | | | KR | 2000035235 | A | 26-06-2000 |
| EP 1095959 | A | 02-05-2001 | JP | 2001122727 | A | 08-05-2001 |
| | | | EP | 1095959 | A2 | 02-05-2001 |
| GB 1120373 | A | 17-07-1968 | BE | 699299 | A | 30-11-1967 |
| | | | NL | 6707586 | A | 01-12-1967 |
| EP 1342731 | A | 10-09-2003 | FR | 2836920 | A1 | 12-09-2003 |
| | | | BR | 0304498 | A | 20-04-2004 |
| | | | EP | 1342731 | A1 | 10-09-2003 |
| | | | JP | 2003267838 | A | 25-09-2003 |
| | | | US | 2004076593 | A1 | 22-04-2004 |

EPO FORM P0460

Pour tout renseignement concernant cette annexe : voir Journal Officiel de l'Office européen des brevets, No.12/82